# EUROPEAN PATENT APPLICATION

(11) **EP 4 793 643 A1**
(43) Date of publication of application: **19.08.2026**
(21) Application number: 23958255.4
(22) Date of filing: 08.11.2023
(51) Int. Cl.: G01N 33/68, G01N 27/447

(54) **INFORMATION PROCESSING SYSTEM, IDENTIFICATION SYSTEM, AND PROGRAM**

(71) Applicant: Aiwell Inc., Tokyo 1050012 (JP); Institute of Science Tokyo, Tokyo 152-8550 (JP)
(72) Inventor: HAYASHI, Nobuhiro, Tokyo 152-8550 (JP); WONG, Sing Ying, Tokyo 152-8550 (JP)
(74) Representative: Strehl & Partner mbB
(86) International application number: PCT/JP2023/040190
(87) International publication number: WO 2025/099851

(57) **Abstract**

An information processing system (1) includes: an acquisition unit (101) configured to acquire molecular information relating to biomolecules of an organism for a plurality of biomolecules; and an identification unit (109) configured to identify a state of the organism according to a target associated with a biomolecule among the plurality of biomolecules, the target satisfying a condition defined for changes in biomolecules identified from the molecular information.

## Description

### TECHNICAL FIELD

The present disclosure relates to an information processing system, an identification system, and a program.

### BACKGROUND

JP 2007-285749 A discloses an influenza infection test reagent and a testing method using a nucleoprotein (NP) of influenza virus as an antigen for detecting AIV antibodies.

### SUMMARY

A technique is known for diagnosing an organism as being in a specific state when a predetermined biomolecule is detected from the organism. However, this technique is unable to identify a state of the organism based on a biomolecule different from the predetermined biomolecule, and is also unable to identify a state of the organism using a plurality of biomolecules.

The present disclosure is directed to identifying a state of an organism using a plurality of biomolecules.

The present disclosure in its first aspect provides an information processing system as specified in claim 1. Optional features are specified in claims 2 to 14.

The present disclosure in its second aspect provides an information processing system as specified in claim 15.

The present disclosure in its third aspect provides an identification system as specified in claim 16.

The present disclosure in its fourth aspect provides a program as specified in claim 17.

Embodiments of the present disclosure enable the identification of a state of an organism using multiple biomolecules.

Features of the present disclosure will become apparent from the following description of embodiments with reference to the appended drawings. The following description of embodiments is described by way of example.

### BRIEF DESCRIPTION OF THE DRAWINGS

FIG. 1 illustrates an example overall configuration of a state identification system.
FIG. 2 illustrates an example hardware configuration of a server device and a terminal.
FIG. 3 illustrates an example functional configuration of the server device.
FIGS. 4A and 4B each illustrate an example organism information management table.
FIG. 5 is a flowchart illustrating a statistics creation process.
FIGS. 6A to 6D illustrate an example sequence until statistical information is created.
FIGS. 7E and 7F illustrate the example sequence until statistical information is created.
FIG. 8 is a flowchart illustrating a state identification process.
FIGS. 9A to 9C illustrate an example sequence until information on a state identified in the state identification process is output to a terminal.
FIGS. 10D and 10E illustrate the example sequence until information on a state identified in the state identification process is output to a terminal.
FIGS. 11F and 11G illustrate the example sequence until information on a state identified in the state identification process is output to a terminal.
FIG. 12 illustrates a state notification screen.
FIGS. 13A and 13B illustrate an example sequence until a state of a subject organism is identified in the state identification process.
FIG. 14C illustrates the example sequence until a state of a subject organism is identified in the state identification process.
FIG. 15 illustrates another example sequence until a state of a subject organism is identified in the state identification process.
FIG. 16 illustrates the example sequence until a state of a subject organism is identified in the state identification process.
FIG. 17 is a flowchart illustrating a state identification process according to a second embodiment.
FIGS. 18A to 18C illustrate images displayed on a display of a terminal when the state identification process according to the second embodiment is performed.
FIGS. 19A and 19B illustrate an example sequence until a state of a subject organism is identified in the state identification process according to the second embodiment.
FIG. 20A illustrates a two-dimensional electrophoresis image showing proteins contained in serum obtained from a horse at a certain time point, and FIG. 20B illustrates the two-dimensional electrophoresis image of FIG. 20A partitioned into a plurality of regions.
FIG. 21A illustrates the body temperature of each subject horse, and FIG. 21B illustrates the amount of amyloid A obtained from each subject horse.
FIG. 22 illustrates statistics calculated for the amount of proteins contained in the serum of each subject horse.

### DESCRIPTION OF THE EMBODIMENTS

### (FIRST EMBODIMENT)

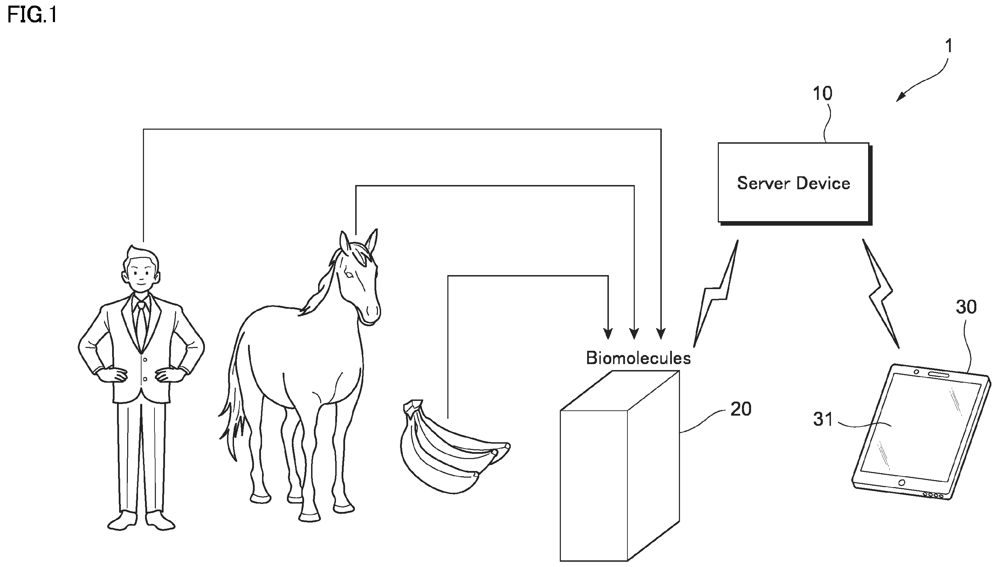

Exemplary embodiments of the present disclosure will be described in detail below with reference to the appended drawings. FIG. 1 illustrates an example overall configuration of a state identification system 1. The state identification system 1, which is an example of the information processing system, is a system for identifying a state of an organism. Examples of the organism include animals, plants, and microorganisms. Examples of the animals include terrestrial animals and aquatic animals. The animals also include humans. The plants also include crops.

Examples of the state of an organism include a health state of the organism, a cosmetic state of the organism, a physical state of the organism when the organism is an animal, a brain state of the organism when the organism is an animal, a quality state of the organism when the organism is food, and a growth state of the organism when the organism is a plant or a microorganism. Examples of the organism as food include crops and animals. Examples of the health state of an organism include a state related to injury or disease of the organism and a state related to fatigue of the organism. Examples of the physical state of an organism include a state related to a height of the organism, a state related to a body weight of the organism, a state related to a body fat percentage of the organism, a state related to a muscle mass of the organism, and a state related to a physical body of the organism. The state related to fatigue of the organism may also be regarded as a physical state of the organism. Examples of the brain state of an organism include a state related to memory of the organism and a state related to thinking ability of the organism. Examples of the quality state of an organism include a state related to taste of the organism. Examples of the growth state of an organism include a state related to a size of each part or the whole of the organism, a state related to a color of the organism, a state related to an odor of the organism, a state related to withering of the organism, and a state related to bacteria or viruses present in the organism.

The state identification system 1 includes a server device 10, a biomolecule analysis device 20, and a terminal 30. The server device 10, the biomolecule analysis device 20, and the terminal 30 are connected via a network.

The server device 10 compares biomolecules of an organism with other biomolecules different from the biomolecules and identifies a state of the organism based on the results of comparison. The organism whose state is to be identified by the server device 10 may be referred to hereinafter as a "subject organism." An organism to be compared with the subject organism by the server device 10 may be referred to hereinafter as a "comparative organism." The server device 10 acquires information indicating physical quantities of biomolecules. Examples of the biomolecules include proteins, amino acids, and peptides. Examples of the physical quantities of biomolecules include their amounts, molecular weights, isoelectric points, and electric charges. The isoelectric point refers to a pH where the average charge of an ionized biomolecule is zero. Information indicating the physical quantities of biomolecules also includes, for example, information indicating a distribution of the physical quantities of biomolecules. Information indicating the physical quantities of biomolecules may be referred to hereinafter as "molecular information." Information indicating the physical quantities of biomolecules of a subject organism may be referred to hereinafter as "subject molecular information." Also, information indicating the physical quantities of biomolecules of a comparative organism may be referred to hereinafter as "comparative molecular information."

Examples of the proteins include hemoglobins, albumins, transferrin, haptoglobin, globulin, lipoproteins, peptide hormones, cytokines, prealbumin, ubiquitin, lactalbumin, glutathione peroxidase, superoxide dismutase, thrombin, prothrombin, actin, acylphosphatase, adrenodoxin, avidin, aldolase, uteroglobin, elastase, ovalbumin, parvalbumin, plastocyanin, relaxin, myoglobin, and leghemoglobin. The proteins may be those contained in body fluids such as blood and saliva. The proteins may be those contained in serum. The proteins may be those contained in a part of an organism. That is, the proteins may be any proteins contained in components of an organism. Also, the proteins may be those different from any of the examples listed above. Examples of the amino acids include valine, isoleucine, leucine, methionine, lysine, phenylalanine, tryptophan, threonine, and histidine. The amino acids may be those contained in body fluids such as blood and saliva. The amino acids may be those contained in serum. The amino acids may be those contained in a part of an organism. That is, the amino acids may be any amino acids contained in components of an organism. Also, the amino acids may be those different from any of the examples listed above.

The server device 10 acquires a plurality of sets of comparative molecular information. The plurality of sets of comparative molecular information are molecular information indicating biomolecules of each comparative organism at different time points. Also, the server device 10 associates respective states of the comparative organism during a period including these time points with the acquired plurality of sets of comparative molecular information. The server device 10 performs, for each comparative organism, the above-described process of acquiring a plurality of sets of comparative molecular information and associating respective states of the corresponding comparative organism with the acquired plurality of sets of comparative molecular information. Thus, the server device 10 stores, for each comparative organism, a plurality of sets of comparative molecular information associated with respective states of the corresponding organism. The server device 10 also acquires a plurality of sets of subject molecular information. The plurality of sets of subject molecular information are molecular information indicating biomolecules of a subject organism at different time points.

Based on the plurality of sets of subject molecular information, the server device 10 detects a change in a biomolecule over time. The server device 10 compares the detected biomolecule with biomolecules shown in the comparative molecular information to thereby extract, from the comparative molecular information stored in the server device 10, comparative molecular information indicating a change in a biomolecule corresponding to the detected change in the biomolecule of the subject organism. The server device 10 then identifies, as a state of the subject organism, a state of the comparative organism associated with the extracted comparative molecular information. Furthermore, the server device 10 outputs information relating to the identified state to the terminal 30.

The server device 10 is implemented by, for example, a computer. The server device 10 may be configured as a single computer, or may be implemented by distributed processing using a plurality of computers. Alternatively, the server device 10 may be implemented on virtual hardware provided by cloud computing services.

The biomolecule analysis device 20 is configured to analyze biomolecules contained in components collected from an organism. More specifically, the biomolecule analysis device 20 analyzes biomolecules contained in components collected from an organism, thereby detecting a distribution of biomolecules. The biomolecule analysis device 20 may be, for example, a two-dimensional electrophoresis device. A two-dimensional electrophoresis device is configured to separate each of the biomolecules contained in components collected from an organism according to molecular weight and isoelectric point, thereby creating a two-dimensional electrophoresis image showing a molecular weight distribution and an isoelectric point distribution of the biomolecules. In the two-dimensional electrophoresis image, for example, positions of pixels constituting spots representing respective biomolecules are defined in a two-dimensional Cartesian coordinate system, in which a distribution of isoelectric points of biomolecules is represented on an x-axis and a distribution of molecular weights of biomolecules is represented on a y-axis. That is, in the two-dimensional electrophoresis image, information indicating whether a biomolecule is present is recorded for each pixel coordinate. The two-dimensional electrophoresis image can be regarded as an example of the molecular information. Hereinafter, information indicating a distribution of biomolecules, such as a two-dimensional electrophoresis image, may be referred to as "distribution information." Also, information indicating a distribution of biomolecules of a subject organism may be referred to as "subject distribution information." Information indicating a distribution of biomolecules of a comparative organism may be referred to hereinafter as "comparative distribution information." The two-dimensional electrophoresis image also includes information indicating a distribution of amounts of biomolecules. That is, the distribution of biomolecules includes a molecular weight distribution of the biomolecules, an isoelectric point distribution of the biomolecules, a distribution of the presence of the biomolecules, and an amount distribution of the biomolecules. Information indicating the amounts of biomolecules in the two-dimensional electrophoresis image may be indicated by, for example, intensity of the spots representing respective biomolecules. For example, a higher intensity of a spot representing a biomolecule in the two-dimensional electrophoresis image may indicate a larger amount of the biomolecule.

The biomolecule analysis device 20 may also be, for example, a biosensor configured to recognize biomolecules in an organism. The biosensor separates the recognized biomolecules by molecular weight and by isoelectric point, thereby creating an image showing a molecular weight distribution and an isoelectric point distribution of the biomolecules. Thus, images created using the biosensor can also be regarded as distribution information. Images created by the biosensor may further include information indicating a distribution of the amounts of biomolecules.

In the present embodiment, a user of the state identification system 1 uses the biomolecule analysis device 20 to analyze components of each comparative organism obtained at a plurality of time points during a certain period, thereby creating comparative distribution information for each of the time points, the information showing a distribution of biomolecules contained in the components. Each of the plurality of time points is a time point at which a state of the comparative organism was identified. The certain period is a period for which a state encompassing the respective states of the comparative organism at the plurality of time points was identified. Each of the states of the comparative organism at the plurality of time points may be referred to hereinafter as an "individual state." The state of the comparative organism encompassing these individual states at the plurality of time points may be referred to hereinafter as an "overall state." Examples of the overall state of the comparative organism include a state of having influenza. For the comparative organism having influenza, examples of the individual states include a state of being infected with the influenza virus and a state of having a fever. Also, the user uses the biomolecule analysis device 20 to create, for each comparative organism, a plurality of sets of comparative distribution information corresponding to different time points at which components of the comparative organism containing the biomolecules were obtained. In this manner, for each comparative organism, a plurality of sets of comparative distribution information are created, each corresponding to one of the time points and showing biomolecules in an individual state at that time point.

In particular, in the present embodiment, the user uses the biomolecule analysis device 20 to analyze, for a plurality of comparative organisms having the same overall state and the same individual states, biomolecules obtained at respective time points corresponding to the individual states, thereby creating comparative molecular information. Also, the user uses the biomolecule analysis device 20 to analyze components of the subject organism obtained at a plurality of time points, thereby creating, for each time point, subject distribution information showing a distribution of biomolecules contained in the components. The plurality of time points are the time points at which a state of the subject organism is to be identified by the state identification system 1.

Upon creating the distribution information, the biomolecule analysis device 20 transmits the created distribution information to the server device 10 together with date and time information indicating the date and time when the biomolecules identified from the distribution information were obtained and with organism information indicating the organism having the biomolecules shown in the distribution information. It should be noted that the organism "having" the biomolecules includes not only an organism that currently has the biomolecules, but also an organism from which the biomolecules have been collected and which no longer has the biomolecules due to the collection.

It should be noted that the biomolecule analysis device 20 is not limited to one configured to analyze biomolecules contained in components collected from an organism. For example, another biomolecule analysis device 20 may be used that is capable of analyzing biomolecules in an organism and creating distribution information without collecting components containing the biomolecules from the organism.

The terminal 30 includes a display 31 for displaying information. Upon acquiring information from the server device 10, the terminal 30 displays the acquired information on the display 31. The terminal 30 is implemented by, for example, a computer, a tablet information terminal, or other information processing devices. The terminal 30 may be, for example, a smartphone. That is, the terminal 30 may be any type of terminal.

The network used for connection between the server device 10, the biomolecule analysis device 20, and the terminal 30 may be any type of network that enables data exchange between them and may be, for example, the Internet, a local area network (LAN), or a wide area network (WAN). The communication line used for data exchange may be either wired or wireless. The devices may also be connected via a plurality of networks or communication lines.

### (Hardware Configuration)

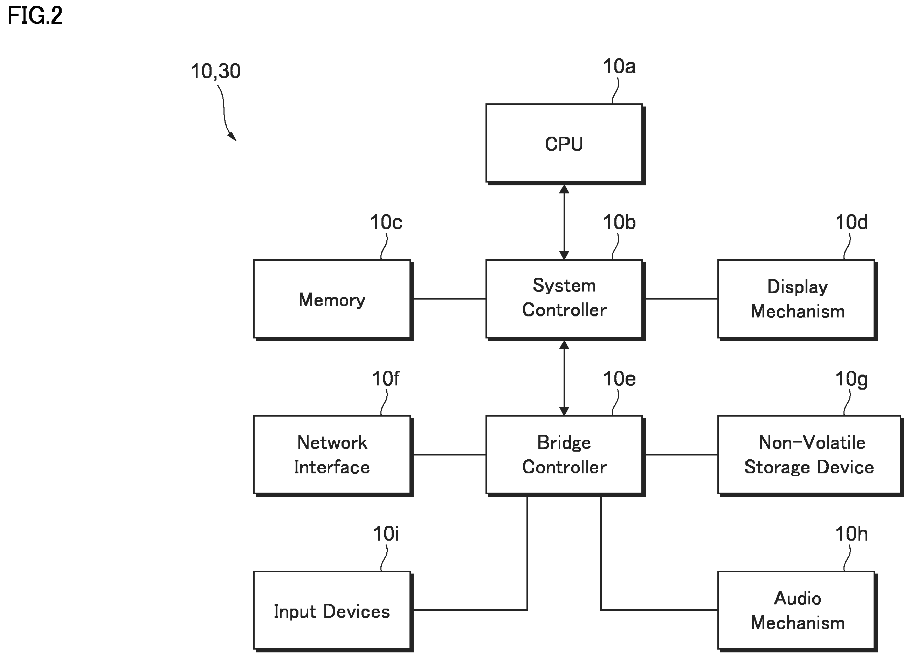

FIG. 2 illustrates an example hardware configuration of the server device 10 and the terminal 30. As shown in FIG. 2, the server device 10 and the terminal 30 each include a central processing unit (CPU) 10a serving as a computing unit and a memory 10c serving as a main memory. The devices 10, 30 each also include external devices such as a non-volatile storage device 10g, a network interface 10f, a display mechanism 10d, an audio mechanism 10h, and input devices 10i such as a keyboard and a mouse.

The memory 10c and the display mechanism 10d are connected to the CPU 10a via a system controller 10b. The network interface 10f, the non-volatile storage device 10g, the audio mechanism 10h, and the input devices 10i are connected to the system controller 10b via a bridge controller 10e. These components are connected by various buses such as a system bus and an input/output bus.

The non-volatile storage device 10g stores a program for implementing various functions. As the program is loaded into the memory 10c and processing based on the program is executed by the CPU 10a, various functions are implemented. Examples of the non-volatile storage device 10g include a semiconductor memory such as a solid state drive (SSD) and a magnetic disk device such as a hard disk drive (HDD).

### (Functional Configuration of the Server Device)

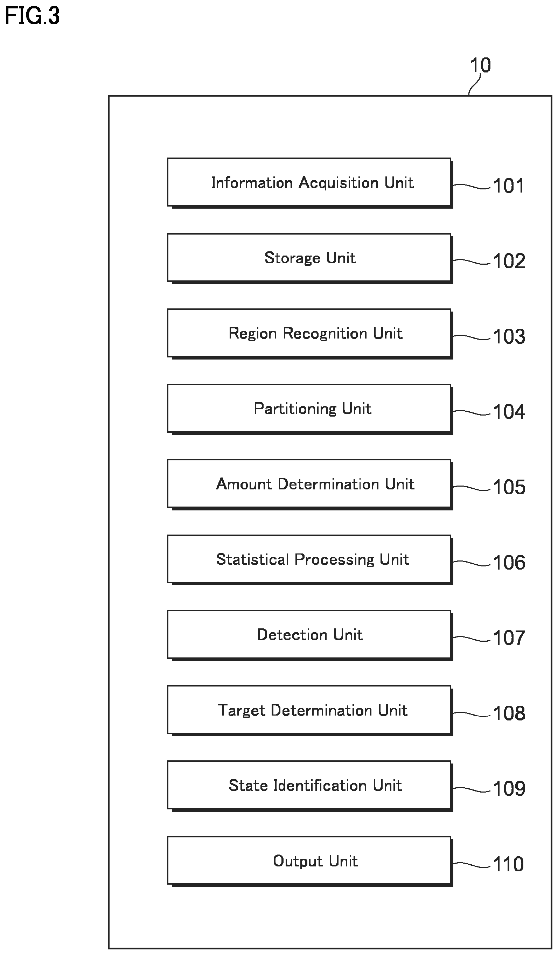

Next, a functional configuration of the server device 10 will be described. FIG. 3 illustrates an example functional configuration of the server device 10. The server device 10 includes an information acquisition unit 101, a storage unit 102, a region recognition unit 103, a partitioning unit 104, an amount determination unit 105, a statistical processing unit 106, a detection unit 107, a target determination unit 108, a state identification unit 109, and an output unit 110.

The information acquisition unit 101, which is an example of the acquisition unit, acquires distribution information from the biomolecule analysis device 20. More specifically, the information acquisition unit 101 acquires distribution information including either subject distribution information or comparative distribution information, together with date and time information and organism information. The information acquisition unit 101 also acquires information indicating a state of each comparative organism. The information indicating a state of each comparative organism may be referred to hereinafter as "state information." Information indicating an individual state of each comparative organism may be referred to hereinafter as "individual state information." Information indicating an overall state of each comparative organism may be referred to hereinafter as "overall state information." A user of the state identification system 1 inputs individual state information corresponding to comparative distribution information into the terminal 30 for each set of comparative distribution information. The individual state information corresponding to comparative distribution information refers to information indicating, among individual states, a state at a time point at which biomolecules shown in the comparative distribution information were obtained. The user also inputs overall state information corresponding to a plurality of sets of comparative distribution information into the terminal 30. The overall state information corresponding to a plurality of sets of comparative distribution information refers to information indicating an overall state for a period including a plurality of time points at which biomolecules shown in the plurality of sets of comparative distribution information were obtained.

Upon acquiring the comparative distribution information and state information, the information acquisition unit 101 stores the acquired state information in the storage unit 102 in association with corresponding comparative distribution information. As described above, in the present embodiment, for a plurality of comparative organisms having the same overall state and the same individual states, comparative distribution information is created for respective time points corresponding to the individual states. Therefore, even for comparative distribution information relating to different comparative organisms, the comparative distribution information is associated with individual state information indicating the same individual states and overall state information indicating the same overall state. The information acquisition unit 101 can also be regarded as the molecular information acquisition unit configured to acquire molecular information.

The storage unit 102 stores the information acquired by the information acquisition unit 101, including the distribution information, date-and-time information, organism information, and state information. Details of contents stored in the storage unit 102 will be described later.

The region recognition unit 103 recognizes one or more regions where biomolecules are present in the distribution information. The region recognition unit 103 recognizes such regions in both subject distribution information and comparative molecular information. An example method used by the region recognition unit 103 to recognize regions where biomolecules are present in distribution information will be described. Specifically, the region recognition unit 103 detects features representing respective biomolecules in the distribution information and identifies coordinates at which the detected features are located, thereby recognizing one or more regions where biomolecules are present. When a plurality of biomolecules are present in the distribution information, the region recognition unit 103 recognizes, for each biomolecule, a region where the biomolecule is present. The region recognition unit 103 recognizes one or more regions where biomolecules are present for each set of distribution information. Upon recognizing one or more regions where biomolecules are present in the distribution information in question, the region recognition unit 103 associates region information indicating the one or more recognized regions with that distribution information.

The partitioning unit 104 partitions the one or more regions where biomolecules are present in the distribution information. More specifically, the partitioning unit 104 partitions the one or more regions where biomolecules are present in the distribution information based on the region information created by the region recognition unit 103. The partitioning unit 104 performs such partitioning for each set of distribution information. The partitioning unit 104 may partition each region where a biomolecule is present in the distribution information as a single region, or may partition that region into a plurality of regions. The partitioning unit 104 may also partition regions where no biomolecules are present in the distribution information. The regions partitioned by the partitioning unit 104 may each be referred to hereinafter as a "partitioned region." The partitioning unit 104 performs such partitioning in both subject distribution information and comparative molecular information.

The amount determination unit 105 determines the amount of each biomolecule shown in the distribution information. More specifically, the amount determination unit 105 determines the amount of biomolecule included in each partitioned region in the distribution information. Upon determining the amount of biomolecule included in each partitioned region in the distribution information, the amount determination unit 105 associates amount information indicating the identified amount with that partitioned region. The amount determination unit 105 determines the amount of biomolecule in both subject distribution information and comparative molecular information.

The statistical processing unit 106 calculates statistics of the amount of biomolecule shown in each set of comparative distribution information associated with the same individual state information. The statistical processing unit 106 extracts, from the comparative distribution information stored in the storage unit 102, comparative distribution information associated with the same individual state information. The statistical processing unit 106 then calculates, for each partitioned region, statistics of the amount of biomolecule included in that partitioned region across the extracted comparative distribution information. More specifically, the statistical processing unit 106 calculates, for each partitioned region having the same coordinates across the extracted comparative distribution information, statistics of the amount of biomolecule included in that partitioned region, based on the amount information associated with that partitioned region. The statistical processing unit 106 calculates statistics of the amount of biomolecule for each set of comparative distribution information associated with the same individual state information. In this manner, statistics of the amount of biomolecule are calculated for each individual state.

The statistical processing unit 106 may calculate statistics of the amount of biomolecule by calculating a mean value of the amount of biomolecule used for statistical calculation. Alternatively, the statistical processing unit 106 may calculate statistics of the amount of biomolecule by calculating a median value of the amount of biomolecule used for statistical calculation. As such, the statistical processing unit 106 may use any suitable statistical method. The statistical processing unit 106 calculates statistics of the amount of biomolecule for each individual state included in an overall state and associates each of the calculated statistics with corresponding individual state information. In this manner, information indicating the statistics calculated by the statistical processing unit 106 is created for each individual state. The information indicating the statistics calculated by the statistical processing unit 106 for each individual state may be referred to hereinafter as "statistical information." The statistical processing unit 106 stores the created statistical information in the storage unit 102 in association with partitioned region information identifying a partitioned region corresponding to the statistical information. Note that the statistical information is information indicating the amount of biomolecule subjected to statistical calculation. Therefore, the statistical information can also be regarded as molecule information in a broad sense.

The detection unit 107 detects a change in a biomolecule of a subject organism. The detection unit 107 compares the amounts of biomolecules shown in two sets of subject distribution information, based on the amount information related to the two sets of subject distribution information. These two sets of subject distribution information indicate biomolecules obtained from the subject organism at respective different time points. Of the two sets of subject distribution information to be compared, the subject distribution information showing biomolecules obtained at earlier time points may be referred to hereinafter as "early-stage distribution information." Of the two sets of subject distribution information to be compared, the subject distribution information showing biomolecules obtained at later time points may be referred to hereinafter as "late-stage distribution information." Biomolecules of the subject organism to be compared are those included in a partitioned region having the same coordinates in the two sets of subject distribution information.

The detection unit 107 determines whether biomolecules to be compared satisfy a change condition. The change condition is a condition defined for detecting changes in biomolecules associated with changes in the state of the subject organism. In the present embodiment, the change condition is defined such that a difference between the amounts of two biomolecules to be compared is greater than or equal to a predetermined value. The detection unit 107 detects two biomolecules satisfying the change condition as biomolecules whose amounts have changed. The two biomolecules detected by the detection unit 107 as satisfying the change condition may each be referred to hereinafter as a "detected biomolecule." The difference between the amounts of the two biomolecules detected by the detection unit 107 may be referred to hereinafter as a "detected value." If biomolecules satisfy the change condition in a plurality of partitioned regions, the detection unit 107 detects all biomolecules in the plurality of partitioned regions.

The target determination unit 108 determines, from the statistical information stored in the storage unit 102, statistical information to be compared with the detected biomolecule. The target determination unit 108 determines, as statistical information to be compared with the detected biomolecule, statistical information relating to a partitioned region having the same coordinates as the partitioned region where the detected biomolecule is present. The target determination unit 108 accesses partitioned region information associated with the statistical information and extracts statistical information relating to a partitioned region having the same coordinates as the partitioned region where the detected biomolecule is present. When detected biomolecules are detected in a plurality of partitioned regions, the target determination unit 108 determines, for each detected biomolecule, statistical information to be used for comparison. The statistical information determined by the target determination unit 108 to be used for comparison with the detected biomolecule may be referred to hereinafter as "comparative statistical information."

The state identification unit 109, which is an example of the identification unit, identifies a state of the subject organism. The state identification unit 109 extracts, from biomolecules shown in comparative statistical information, one or more biomolecules satisfying a sameness/similarity condition. The sameness/similarity condition is a condition defined for identifying a change in a biomolecule that is identical or similar to the change in the amount of biomolecule indicated by the detected value for the detected biomolecule. In the present embodiment, the sameness/similarity condition is defined such that a change in the amount of biomolecule shown in the comparative statistical information is identical or similar to the change in the amount of biomolecule indicated by the detected value. For example, the sameness/similarity condition may be such that a difference between a change in the amount of biomolecule shown in the comparative statistical information and the detected value is less than or equal to a predetermined value. The state identification unit 109 identifies, as a state of the subject organism, an overall state associated with the comparative statistical information satisfying the sameness/similarity condition. The comparative statistical information satisfying the sameness/similarity condition refers to comparative statistical information indicating a biomolecule satisfying the sameness/similarity condition.

The output unit 110, which is an example of the output unit, outputs information relating to the state identified by the state identification unit 109 to the terminal 30.

### (Contents Stored in Storage Unit)

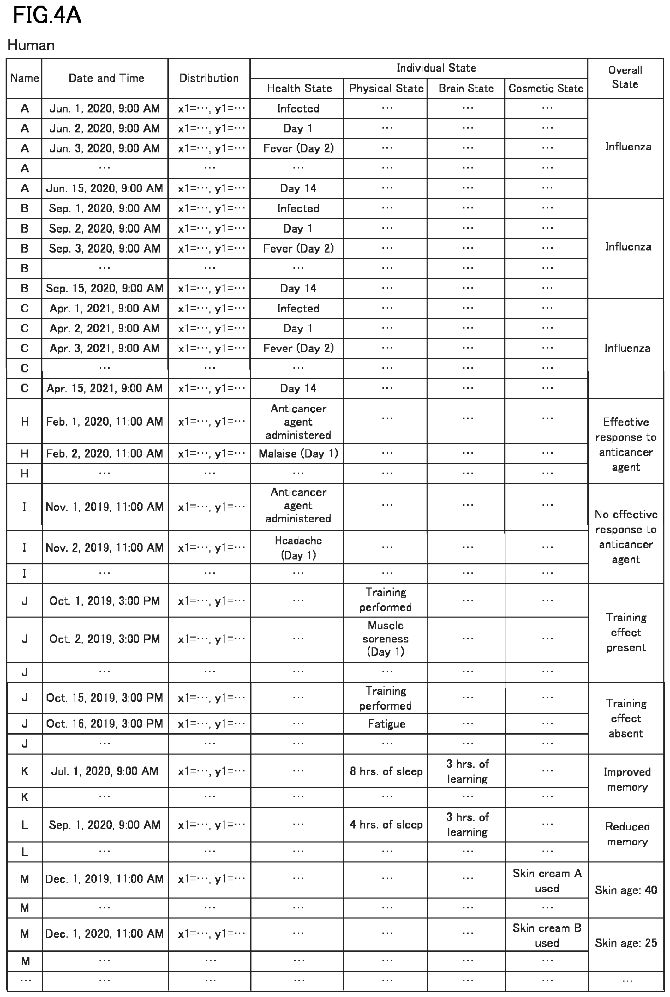

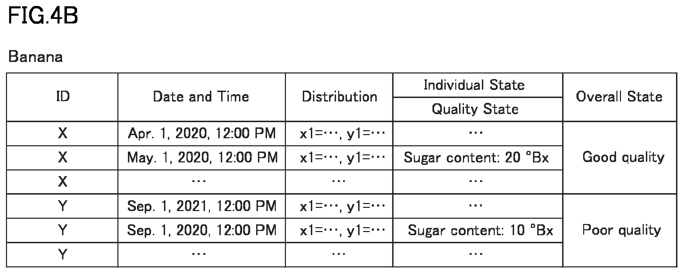

Contents of information stored in the storage unit 102 will now be described. FIGS. 4A and 4B each illustrate an example organism information management table. The organism information management table is a table for managing information on comparative organisms. The organism information management table is provided for each type of organism. FIG. 4A shows an organism information management table for "human," which is a type of animal, and FIG. 4B shows an organism information management table for "banana," which is a type of crop.

In the organism information management table of FIG. 4A, the column entitled "Name" lists the names of comparative organisms. The column entitled "Date and Time" lists the date and time information. The column entitled "Distribution" lists the comparative distribution information. More specifically, the column "Distribution" lists the distributions of physical quantities of biomolecules identified from the distribution information.

The column entitled "Individual State" lists the individual state information. In the present embodiment, the column "Individual State" is divided into four subcolumns: "Health State," "Physical State," "Brain State," and "Cosmetic State." The subcolumn "Health State" lists the health states of the comparative organisms. The subcolumn "Physical State" lists the physical states of the comparative organisms. The subcolumn "Brain State" lists the brain states of the comparative organisms. The subcolumn "Cosmetic State" lists the cosmetic states of the comparative organisms. The column "Individual State" may also include information relating to the growth states of the comparative organisms. The column entitled "Overall State" lists the overall state information.

The information shown in the columns "Individual State" and "Overall State" in the organism information management table of FIG. 4A will be described in detail. For example, comparative distribution information of organism "A" is associated with "influenza" in the column "Overall State" and with "infected," "fever," and other information in the column "Health State". The overall state "Influenza" is a state in which the comparative organism has influenza. The health state "infected" is a state in which the comparative organism is infected with an influenza virus, and the health state "fever" is a state in which the comparative organism has a fever. Also, for example, comparative distribution information of organism "H" is associated with "effective response to anticancer agent" in the column "Overall State" and with "anticancer agent administered," "malaise," and other information in the column "Health State." The overall state "effective response to anticancer agent" is a state in which the comparative organism exhibits an effect resulting from administration of an anticancer agent. The health state "anticancer agent administered" is a state in which an anticancer agent has been administered to the comparative organism, and the health state "malaise" is a state in which the comparative organism has malaise. Also, for example, comparative distribution information of organism "I" is associated with "no effective response to anticancer agent" in the column "Overall State" and with "anticancer agent administered," "headache," and other information in the column "Health State". The overall state "no effective response to anticancer agent" is a state in which the comparative organism exhibits no effect resulting from administration of an anticancer agent. The health state "headache" is a state in which the comparative organism has a headache.

Also, for example, comparative distribution information of organism "J" is associated with "training effect present" in the column "Overall State" and with "training performed," "muscle soreness," and other information in the column "Physical State." The overall state "training effect present" is a state in which the comparative organism exhibits an effect resulting from performing training. The physical state "training performed" is a state in which the comparative organism has performed training. The physical state "muscle soreness" is a state in which the comparative organism experiences muscle soreness. For example, the comparative distribution information of organism "J" is also associated with "training effect absent" in the column "Overall State" and with "training performed," "fatigue," and other information in the column "Physical State." The overall state "training effect absent" is a state in which the comparative organism exhibits no effect resulting from performing training. The physical state "fatigue" is a state in which the comparative organism has fatigue. Also, for example, comparative distribution information of organism "K" is associated with "improved memory" in the column "Overall State" and with "8 hours of sleep" and other information in the column "Physical State" and "3 hours of learning" and other information in the column "Brain State." The overall state "improved memory" is a state in which memory of the comparative organism has improved. The physical state "8 hours of sleep" is a state in which the comparative organism has slept for 8 hours, and the brain state "3 hours of learning" is a state in which the comparative organism has engaged in learning for 3 hours. Also, for example, comparative distribution information of organism "L" is associated with "reduced memory" in the column "Overall State" and with "4 hours of sleep" and other information in the column "Physical State" and "3 hours of learning" and other information in the column "Brain State." The overall state "reduced memory " is a state in which memory of the comparative organism has declined. The physical state "4 hours of sleep" is a state in which the comparative organism has slept for 4 hours.

Also, for example, comparative distribution information of organism "M" is associated with "skin age: 40" in the column "Overall State" and with "skin cream A used" and other information in the column "Cosmetic State." The overall state "skin age: 40" is a state in which a skin age of the comparative organism is determined to be 40. The cosmetic state "skin cream A used" is a state in which the comparative organism has used "skin cream A." For example, the comparative distribution information of organism "M" is also associated with "skin age: 25" in the column "Overall State" and with "skin cream B used" and other information in the column "Cosmetic State." The overall state "skin age: 25" is a state in which a skin age of the comparative organism is determined to be 25. The cosmetic state "skin cream B used" is a state in which the comparative organism has used "skin cream B."

In the organism information management table of FIG. 4B, the column entitled "ID" lists the names for identifying respective comparative organisms. In the illustrated example, the column "ID" lists the names for identifying respective bananas. The column entitled "Date and Time" lists the date and time information. The column entitled "Distribution" lists the comparative distribution information, as in the example shown in FIG. 4A. The column entitled "Individual State" lists the individual state information. In the present embodiment, the column "Individual State" shows "Quality State," which indicates the quality state of each comparative organism. The column entitled "Overall State" lists the overall state information.

The information shown in the columns "Individual State" and "Overall State" in the organism information management table of FIG. 4B will be described in detail. Comparative distribution information of organism "X" is associated with "good quality" in the column "Overall State" and with "sugar content: 20 °Bx" and other information in the column "Quality State." The overall state "good quality" is a state in which the quality of the comparative organism is determined to be good, and the quality state "sugar content: 20 °Bx" is a state in which the sugar content of the comparative organism is determined to be 20 °Bx. Also, comparative distribution information of organism "Y" is associated with "poor quality" in the column "Overall State" and with "sugar content: 10 °Bx" and other information in the column "Quality State." The overall state "poor quality" is a state in which the quality of the comparative organism is determined to be poor, and the quality state "sugar content: 10 °Bx" is a state in which the sugar content of the comparative organism is determined to be 10 °Bx. Although not shown in FIGS. 4A and 4B, the storage unit 102 stores an organism information management table for each type of comparative organism.

### (Statistics Creation Process)

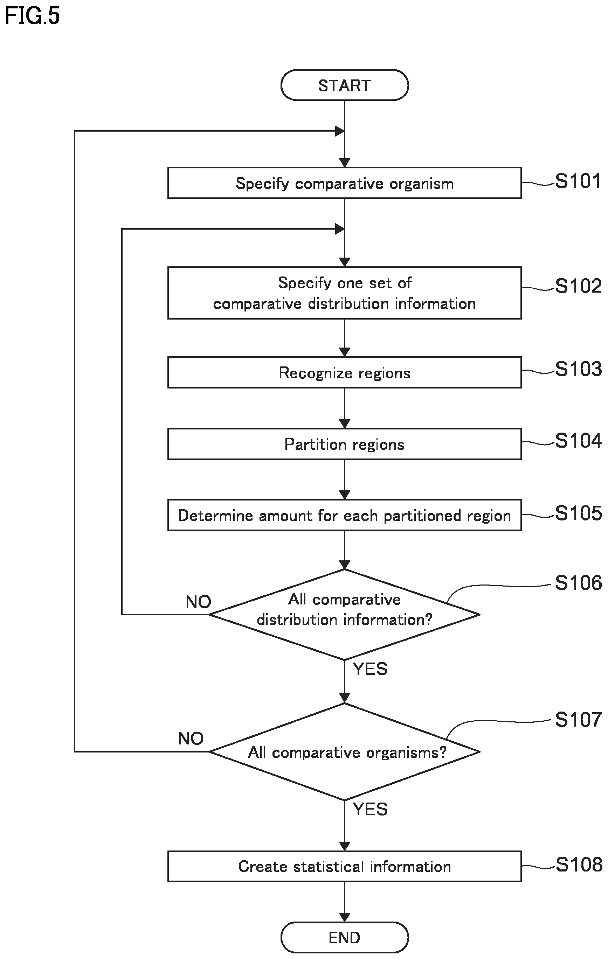

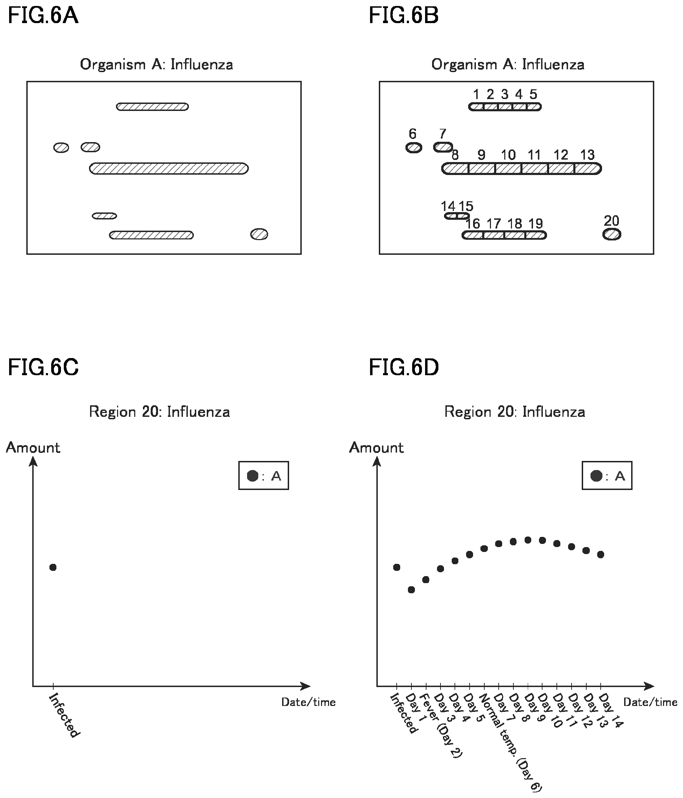

A flow of a statistics creation process will now be described with reference to an example sequence until statistical information is created. The statistics creation process is a process in which the server device 10 creates statistical information. In the present embodiment, the statistics creation process is initiated in response to a user of the state identification system 1 specifying an overall state and instructing the server device 10 to execute the statistics creation process. The overall state specified by the user may be referred to hereinafter as a "specified state." The following describes a case in which the specified state is "influenza." FIG. 5 is a flowchart illustrating the statistics creation process. FIGS. 6A to 7F illustrate an example sequence until statistical information is created.

First, the server device 10 specifies a comparative organism among comparative organisms that were in the specified state (step (hereinafter "S") 101). The server device 10 identifies one or more comparative organisms that were in the specified state based on organism information associated with overall state information indicating the specified state. The comparative organisms specified by the server device 10 in step 101 may each be referred to hereinafter as a "specified organism." Then, the server device 10 specifies one set of comparative distribution information associated with the overall state information indicating the specified state (S102). The comparative distribution information specified by the server device 10 in step 102 is comparative distribution information indicating biomolecules of the specified organism. The comparative distribution information specified by the server device 10 in step 102 may be referred to hereinafter as "specified distribution information."

In this example, the specified state is "influenza," as described above. In this case, if the specified organism is organism "A," comparative distribution information relating to organism "A" and associated with "influenza" is specified in step102, as shown in FIG. 6A. The specified distribution information shown in FIG. 6A is one set of comparative distribution information included in the organism information management table for "Human" (see FIG. 4A) and associated with organism "A" and the overall state "influenza." This specified distribution information is information showing a distribution of the amount of biomolecule, with the isoelectric point of biomolecules represented on the horizontal axis in the figure and the molecular weight of biomolecules represented on the vertical axis in the figure. In the specified distribution information shown in FIG. 6A, each image indicated by hatching and a lines surrounding the hatching represents a biomolecule. In the distribution information shown in FIG. 6A, seven mutually different biomolecules are shown. Note that while the biomolecules are spaced apart from each other in the distribution information in the illustrated example, a plurality of biomolecules may be adjacent to each other in the distribution information.

Next, the region recognition unit 103 recognizes one or more regions where biomolecules are present in the specified distribution information (S103). The partitioning unit 104 partitions the one or more regions recognized by the region recognition unit 103 in the specified distribution information (S104). In this example, in step 104, the partitioning unit 104 partitions the regions showing the respective biomolecules in the specified distribution information into twenty regions consisting of Region 1 to Region 20, as shown in FIG. 6B. In the example shown in FIG. 6B, one biomolecule is partitioned into five regions consisting of Region 1 to Region 5. Another biomolecule is partitioned into one region consisting of Region 6. Another biomolecule is partitioned into one region consisting of Region 7. Another biomolecule is partitioned into six regions consisting of Region 8 to Region 13. Another biomolecule is partitioned into two regions consisting of Region 14 and Region 15. Another biomolecule is partitioned into four regions consisting of Region 16 to Region 19. Another biomolecule is partitioned into one region consisting of Region 20.

Next, the amount determination unit 105 determines, for each partitioned region, the amount of biomolecule included in that partitioned region (S105). In this example, in step 105, the amount determination unit 105 determines the amount of biomolecule included in each partitioned region as shown in FIG. 6C. FIG. 6C illustrates a biomolecule present in Region 20, which is one of the partitioned regions, where the horizontal axis in the figure represents the time point at which the biomolecule was obtained and the vertical axis in the figure represents the amount of biomolecule. In FIG. 6C, the symbol of a black circle indicates the biomolecule of organism "A". Further, the biomolecule shown in FIG. 6C is associated with "infection," which is one of the individual states. This state "infection" is one of the individual states (see FIG. 4A) associated with the comparative distribution information showing the biomolecules of organism "A" identified in FIG. 6C. Note that while only the biomolecule in Region 20 is shown in the example of FIG. 6C, in step 105, the amount of biomolecule is determined for each of Regions 1 to 20, that is, for all partitioned regions in the specified distribution information.

Next, the server device 10 determines whether the amount of biomolecule has been determined for all sets of comparative distribution information relating to the specified organism and associated with the overall state information indicating the specified state (S106). If the amount of biomolecule has not been determined for any one of the sets of comparative distribution information associated with the overall state information indicating the specified state (NO in S106), the server device 10 newly specifies one set of comparative distribution information for which the amount has not been determined (S102). Following this, step 103 and subsequent steps are performed.

In this manner, step 102 and subsequent steps are repeated until the amount of biomolecule is determined for all sets of comparative distribution information relating to the specified organism and associated with the overall state information indicating the specified state. As a result, in this example, the amount of biomolecule present in each partitioned region is determined for all sets of comparative distribution information associated with the state "influenza" specified for organism "A," as shown in FIG. 6D. FIG. 6D illustrates the biomolecule present in Region 20 at each time point at which the biomolecule was obtained during the period in which the specified organism was identified as being in the state of "influenza," where the horizontal axis represents the time point at which the biomolecule was obtained and the vertical axis represents the amount of biomolecule. In FIG. 6D, plots of the amount of biomolecule are each associated with an individual state. This individual state is one of the individual states (see FIG. 4A) associated with the comparative distribution information indicating the biomolecules of organism "A" identified in FIG. 6D. Further, these individual states are labeled with the number of days from "Day 1" to "Day 14." This number of days indicates the number of days elapsed since organism "A" was infected with an influenza virus. Note that while only the biomolecule in Region 20 is shown in the example of FIG. 6D, the amount of biomolecule in all partitioned regions is determined by repeating steps 102 to 106.

Once the amount of biomolecule is determined for all sets of comparative distribution information relating to the specified organism and associated with the overall state information indicating the specified state (YES in S106), the process proceeds to the next step. The server device 10 determines whether the amount of biomolecule in the comparative distribution information has been determined for all comparative organisms that were in the specified state (S107). If the amount of biomolecule in the comparative distribution information has not been determined for any one of the comparative organisms that were in the specified state (NO in S107), the server device 10 newly specifies one comparative organism for which the amount of biomolecule in the comparative distribution information has not been determined (S101). Following this, step 102 and subsequent steps are performed.

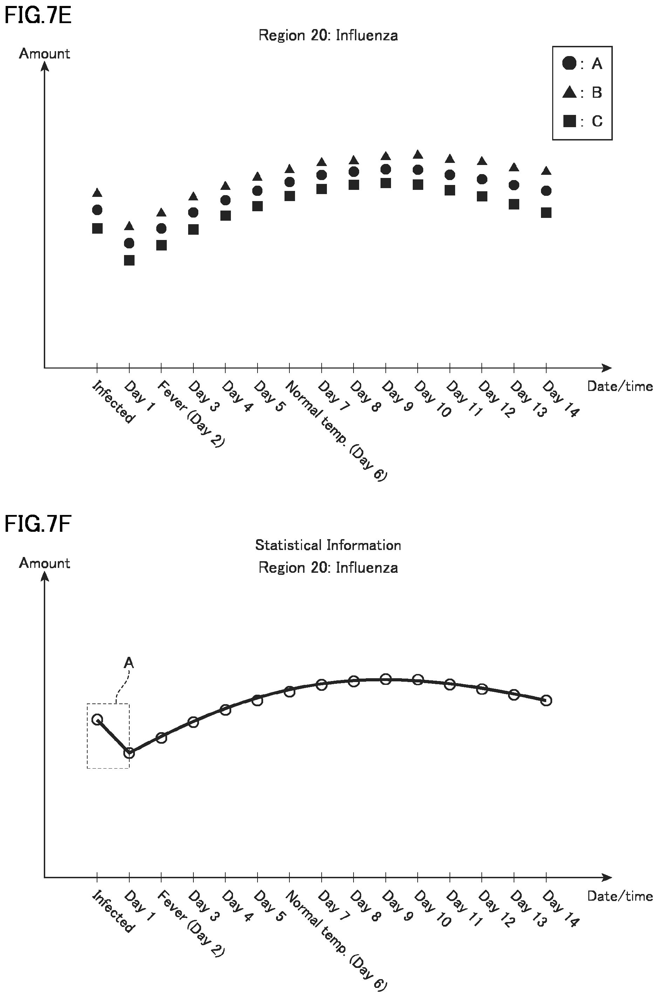

In this manner, step 101 and subsequent steps are repeated until the amount of biomolecule in the comparative distribution information is determined for all comparative organisms that were in the specified state. As a result, in this example, the amount of biomolecule present in each partitioned region is determined for all sets of comparative distribution information associated with "influenza" for organisms "A," "B," and "C," which are all comparative organisms that were in the specified state, as shown in FIG. 7E. FIG. 7E illustrates plots of the amount of biomolecule present in Region 20 at each time point at which the biomolecule was obtained during the period in which each of the organisms "A," "B," and "C" was identified as being in the state of "influenza." In FIG. 7E, the horizontal axis represents the time point at which the biomolecule was obtained, and the vertical axis represents the amount of biomolecule. In FIG. 7E, the symbols of a black circle each indicate the biomolecule of organism "A," the symbols of a black triangle each indicate the biomolecule of organism "B," and the symbols of a black square each indicate the biomolecule of organism "C." Furthermore, the amounts of biomolecule of organisms "A," "B," and "C" are each associated with the same individual state information. This individual state is one of the individual states (see FIG. 4A) associated with the comparative distribution information showing the respective biomolecules of organisms "A," "B," and "C" identified in FIG. 7E. While only the biomolecule in Region 20 is shown in the example of FIG. 7E, the amount of biomolecule in all partitioned regions is determined by repeating steps 101 to 107.

Once the amount of biomolecule in comparative distribution information is determined for all comparative organisms that were in the specified state (YES in S107), the statistical processing unit 106 calculates statistics of the amount of biomolecule associated with the same state among the individual states. More specifically, the statistical processing unit 106 calculates, for each individual state, statistics of the amount of biomolecule associated with the same state among the individual states, thereby creating statistical information indicating statistics of the amount of biomolecule for each individual state (S108). The statistical processing unit 106 creates such statistical information for each partitioned region. In this example, the statistical processing unit 106 calculates, for each individual state, statistics of the amount of biomolecule of organisms "A," "B," and "C" associated with the same state among the individual states, thereby creating statistical information shown in FIG. 7F. FIG. 7F illustrates statistics of the amount of biomolecule in Region 20. This statistical information shows, for each individual state, statistics of the amount of biomolecule of organisms "A," "B," and "C".

### (State Identification Process)

A flow of a state identification process will now be described with reference to an example sequence until information on the state identified in the state identification process is output to the terminal 30. The state identification process is a process in which the server device 10 identifies a state of a subject organism. In the present embodiment, the state identification process is initiated in response to a user of the state identification system 1 specifying subject distribution information used for the state identification process and instructing the server device 10 to execute the state identification process. Hereinafter, subject distribution information specified by the user as the subject distribution information used for the state identification process may be referred to as "used distribution information."

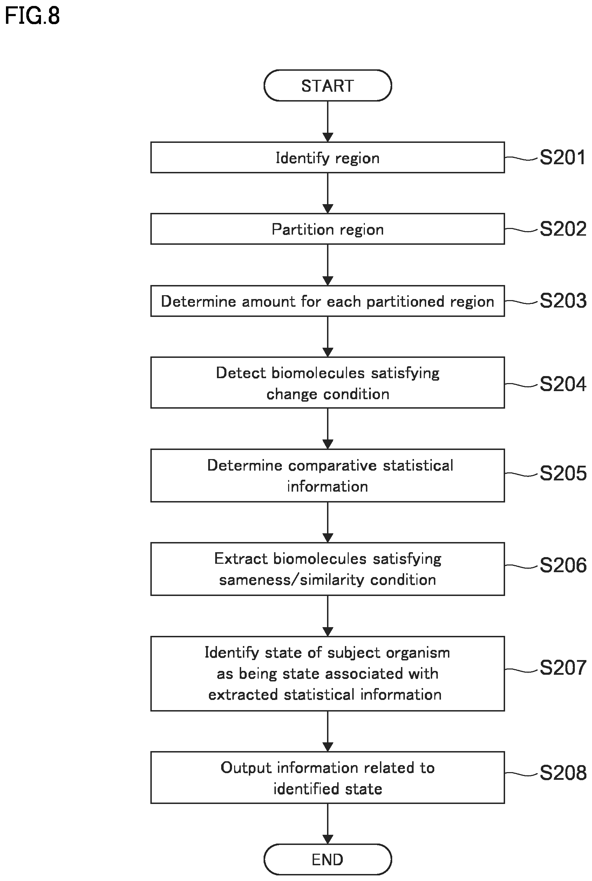

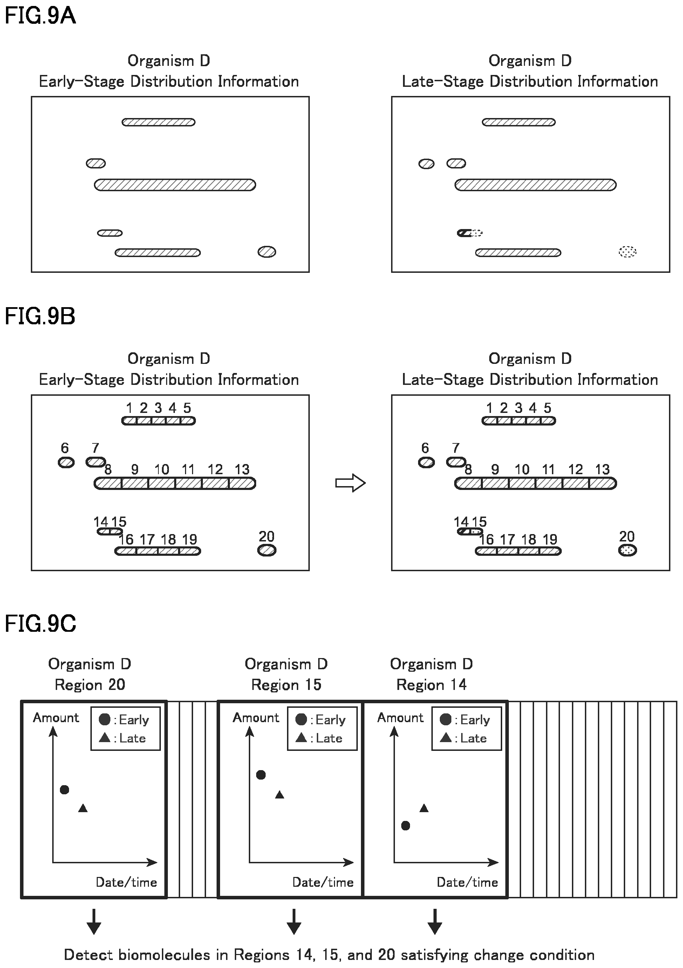

FIG. 8 is a flowchart illustrating the state identification process. FIGS. 9A to 12 illustrate an example sequence until information on the state identified in the state identification process is output to the terminal 30. The following describes a case where the subject organism is organism "D" and the used distribution information consists of two sets of subject distribution information shown in FIG. 9A. The used distribution information shown in FIG. 9A includes early-stage distribution information and late-stage distribution information for organism "D". The early-stage distribution information and the late-stage distribution information each show a distribution of seven mutually different biomolecules, as in the distribution information shown in FIG. 6A. Organism "D" is, for example, a human.

First, for each set of used distribution information, the region recognition unit 103 recognizes one or more regions where biomolecules are present in the used distribution information (S201). The partitioning unit 104 partitions each region recognized by the region recognition unit 103 for each set of used distribution information (S202). In this example, in step 202, the partitioning unit 104 partitions the regions showing the respective biomolecules in each of the early-stage distribution information and the late-stage distribution information into twenty regions consisting of Region 1 to Region 20, as shown in FIG. 9B. That is, the partitioning unit 104 partitions the regions showing the respective biomolecules in the used distribution information in the same manner as the partitioning performed on the comparative distribution information (see FIG. 6B).

Next, for each set of used distribution information, the amount determination unit 105 determines the amount of biomolecule included in each partitioned region (S203). As a result, in this example, the amount of biomolecule is determined for each partition region in each of the early-stage distribution information and the late-stage distribution information for organism "D," as shown in FIG. 9C. FIG. 9C illustrates the biomolecule present in each region across the early-stage distribution information and the late-stage distribution information, where the horizontal axis in the figure represents the time point at which the biomolecule was obtained and the vertical axis in the figure represents the amount of biomolecule. In FIG. 9C, the symbols of a black circle each indicate the biomolecule shown in the early-stage distribution information, and the symbols of a black triangle each indicate the biomolecule shown in the late-stage distribution information.

Next, the detection unit 107 detects one or more biomolecules that satisfy the change condition (S204). More specifically, the detection unit 107 determines, for each partitioned region, whether a biomolecule satisfies the change condition, thereby detecting one or more biomolecule determined to satisfy the change condition. In this example, in each of Regions 14, 15, and 20 shown in FIG. 9C, the amount of biomolecule shown in the late-stage distribution information has changed from that shown in the early-stage distribution information. More specifically, in Region 14, the amount of biomolecule shown in the late-stage distribution information is greater than that shown in the early-stage distribution information. In each of Regions 15 and 20, the amount of biomolecule shown in the late-stage distribution information is less than that shown in the early-stage distribution information. Thus, the biomolecules included in Regions 14, 15, and 20 are each detected by the detection unit 107 as satisfying the change condition.

Note that the plurality of biomolecules satisfying the change condition, that is, the biomolecules included in Regions 14 and 15 and the biomolecule included in Region 20, are different biomolecules. Examples of such different biomolecules include hemoglobin and transferrin. Other examples of such different biomolecules include hemoglobin HbA and hemoglobin HbA2. That is, the different biomolecules may be such that the molecules constituting the biomolecules are not identical.

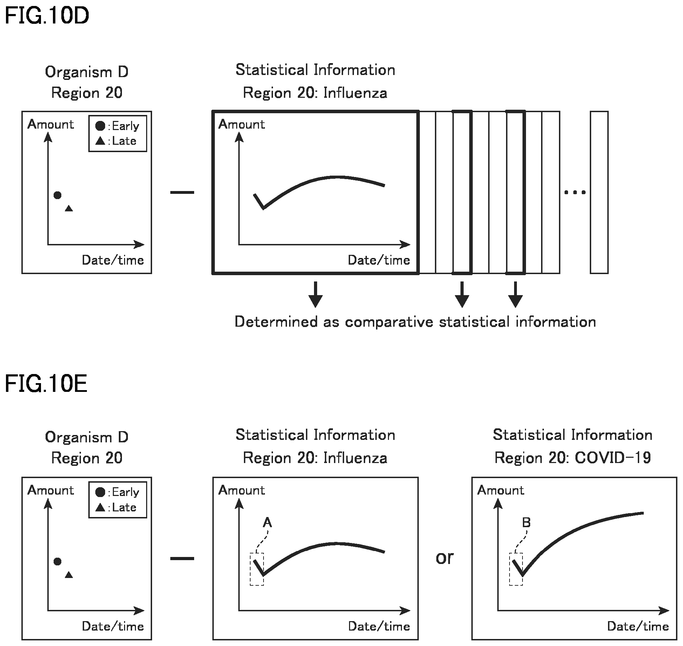

Next, the target determination unit 108 determines comparative statistical information to be compared with each detected biomolecule (S205). The target determination unit 108 determines, as the comparative statistical information, statistical information corresponding to a partitioned region having the same coordinates as the partitioned region where the detected biomolecule is present, among statistical information stored in the storage unit 102 . In this example, the target determination unit 108 determines each set of statistical information for Region 20 as the comparative statistical information corresponding to the detected biomolecule shown in Region 20 for organism "D". As shown in FIG. 10D, this comparative statistical information includes statistical information for Region 20 associated with "influenza" as an overall state. Although not shown in the figures, the target determination unit 108 also determines each set of statistical information for Region 15 as the comparative statistical information corresponding to the detected biomolecule shown in Region 15 for organism "D". The target determination unit 108 also determines each set of statistical information for Region 14 as the comparative statistical information corresponding to the detected biomolecule shown in Region 14 for organism "D".

Next, the state identification unit 109 extracts, from biomolecules shown in the comparative statistical information, one or more biomolecules satisfying the sameness/similarity condition (S206). More specifically, the state identification unit 109 compares each detected biomolecule with corresponding biomolecules shown in the comparative statistical information, thereby extracting one or more biomolecules satisfying the sameness/similarity condition from the biomolecules shown in the comparative statistical information. In this example, as shown in FIG. 10E, the state identification unit 109 determines that biomolecules shown in two sets of comparative statistical information satisfy the sameness/similarity condition with respect to the detected biomolecule included in Region 20 for organism "D". One of the two sets of comparative statistical information is comparative statistical information for Region 20 associated with "influenza" as an overall state. In this comparative statistical information, the dashed-line portion A in the figure indicates a decrease in biomolecule amount over time, and the state identification unit 109 determines that the biomolecule shown in this portion A satisfies the sameness/similarity condition with respect to the detected biomolecules included in Region 20 for organism "D". Note that this comparative statistical information for Region 20 associated with "influenza" is the statistical information shown in FIG. 7F. The other of the two sets of comparative statistical information is comparative statistical information for Region 20 associated with "Corona Virus Disease (COVID-19)" as an overall state. In this comparative statistical information as well, the dashed-line portion B in the figure indicates a decrease in biomolecule amount over time, and the state identification unit 109 determines that the biomolecule shown in this portion B satisfies the sameness/similarity condition with respect to the detected biomolecules included in Region 20 for organism "D".

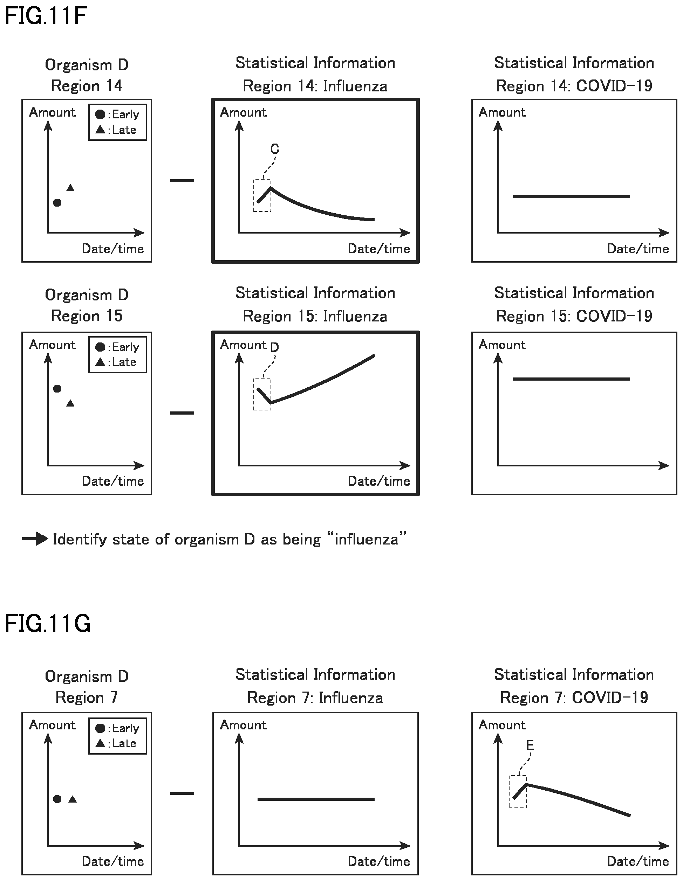

As described above, there may be cases where biomolecules shown in a plurality of sets of comparative statistical information are all determined to satisfy the sameness/similarity condition with respect to a detected biomolecule included in a partitioned region. To further illustrate, influenza virus and COVID-19 are both infectious diseases, and thus a specific biomolecule in the subject organism may exhibit the same or similar change regardless of whether the subject organism is infected with the influenza virus or COVID-19. In this case, the state identification unit 109 compares one or more biomolecules shown in the extracted comparative statistical information with the detected biomolecule included in another partitioned region for which comparison has not yet been performed. In this example, as shown in FIG. 11F, the state identification unit 109 compares the detected biomolecule shown in Region 14 for organism "D" with a corresponding biomolecule shown in comparative statistical information for Region 14 associated with "influenza." The state identification unit 109 also compares this detected biomolecule shown in Region 14 for organism "D" with a corresponding biomolecule shown in comparative statistical information for Region 14 associated with "COVID-19." Furthermore, the state identification unit 109 compares the detected biomolecule shown in Region 15 for organism "D" with a corresponding biomolecule shown in comparative statistical information for Region 15 associated with "influenza." The state identification unit 109 also compares this detected biomolecule shown in Region 15 for organism "D" with a corresponding biomolecule shown in comparative statistical information for Region 15 associated with "COVID-19."

In the comparative statistical information for Region 14 associated with "influenza," the dashed-line portion C in the figure indicates an increase in biomolecule amount over time. Thus, the state identification unit 109 determines that the biomolecule shown in this portion C satisfies the sameness/similarity condition with respect to the detected biomolecule shown in Region 14 for organism "D". Also, in the comparative statistical information for Region 15 associated with "influenza," the dashed-line portion D in the figure indicates a decrease in biomolecule amount over time. Thus, the state identification unit 109 determines that the biomolecule shown in this portion D satisfies the sameness/similarity condition with respect to the detected biomolecule shown in Region 15 for organism "D". On the other hand, the comparative statistical information for Region 14 associated with "COVID-19" indicates no change in biomolecule amount over time. Thus, the state identification unit 109 determines that this biomolecule does not satisfy the sameness/similarity condition with respect to the detected biomolecule shown in Region 14 for organism "D". Also, the comparative statistical information for Region 15 associated with "COVID-19" indicates no change in biomolecule amount over time. Thus, the state identification unit 109 also determines that this biomolecule does not satisfy the sameness/similarity condition with respect to the detected biomolecule shown in Region 15 for organism "D". In this case, the state identification unit 109 extracts the biomolecule shown in each statistical information associated with "influenza" as a biomolecule satisfying the sameness/similarity condition. The comparative statistical information showing the biomolecule extracted by the state identification unit 109 as satisfying the sameness/similarity condition may be referred to hereinafter as "extracted statistical information."

Next, the state identification unit 109 identifies a state of the subject organism based on the extracted statistical information. More specifically, the state identification unit 109 identifies, as a state of the subject organism, an overall state associated with the extracted statistical information (S207). The state of the subject organism identified by the state identification unit 109 may be referred to hereinafter as an "identified state." In this example, the state identification unit 109 identifies organism "D" as being in the state of "influenza," which is an overall state associated with the extracted statistical information.

In this example, a biomolecule shown in the comparative statistical information associated with "influenza" and a biomolecule shown in the comparative statistical information associated with "COVID-19" both satisfy the sameness/similarity condition with respect to the detected biomolecule included in Region 20.

FIG. 11G illustrates the biomolecule included in Region 7 in the early-stage distribution information and the late-stage distribution information, as well as comparative statistical information for Region 7 associated with "influenza" and comparative statistical information for Region 7 associated with "COVID-19." In each of the diagrams shown in FIG. 11G, the horizontal axis represents the time point at which the biomolecule was obtained and the vertical axis represents the amount of biomolecule. In FIG. 11G, the symbol of a black circle indicates the biomolecule shown in the early-stage distribution information, and the symbol of a black triangle indicates the biomolecule shown in the late-stage distribution information. As shown in FIG. 11G, in Region 7 for organism "D," there is no change in biomolecule amount between the early-stage distribution information and the late-stage distribution information. There is also no change in the biomolecule amount over time in the comparative statistical information for Region 7 associated with "influenza." On the other hand, in the comparative statistical information for Region 7 associated with "COVID-19," the dashed-line portion E in the figure indicates an increase in biomolecule amount over time. Therefore, when the biomolecule of organism "D" in Region 20 exhibits a change like at the portion B in FIG. 10E and the biomolecule of organism "D" in Region 7 exhibits a change like that at the portion E in FIG. 11G, the state identification unit 109 identifies a state of organism "D" as being "COVID-19."

In the present embodiment, the state identification unit 109 identifies a state of the subject organism as being "influenza" in response to biomolecules shown in the comparative statistical information associated with "influenza" satisfying the sameness/similarity condition with respect to all of the detected biomolecules included in Regions 20, 15, and 14. Here, there may be cases where biomolecules shown in the comparative statistical information associated with "influenza" satisfy the sameness/similarity condition with respect to the detected biomolecules included in Regions 20 and 15 but do not satisfy the sameness/similarity condition with respect to the detected biomolecule included in Region 14. In this case, the state identification unit 109 may identify a state of the subject organism as being a state different from "influenza."

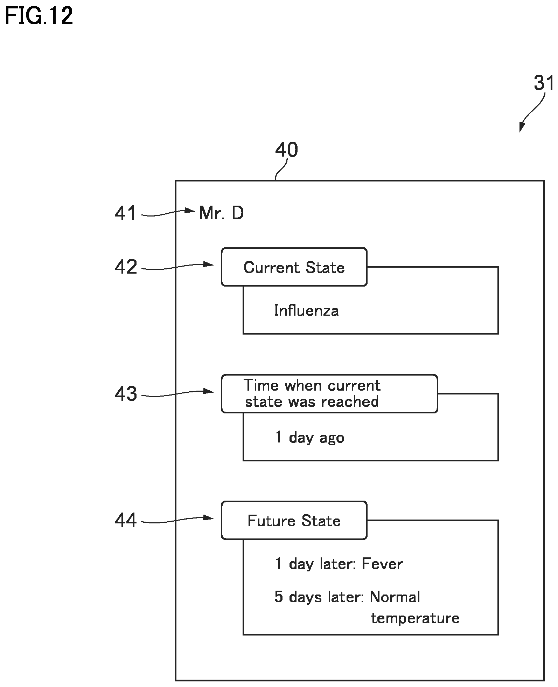

Next, the output unit 110 outputs information on the identified state to the terminal 30 (S208). In this example, the output unit 110 causes a state notification screen 40 to be displayed on the display 31 of the terminal 30, as shown in FIG. 12. The state notification screen 40 is a screen for notifying a user of the identified state. The state notification screen 40 includes an organism identification section 41, a state notification section 42, a timing notification section 43, and a future state notification section 44. Hereinafter, the date on which the state notification screen 40 is displayed is assumed to be the same as the date on which the biomolecules shown in the late-stage distribution information were obtained.

The organism identification section 41 shows information for identifying the subject organism. In the illustrated example, the organism identification section 41 shows text "Mr. D" including information for identifying the subject organism. The state notification section 42 shows the identified state. In the illustrated example, the state notification section 42 shows text "Current State" and text "Influenza," which is the identified state.

The timing notification section 43 shows the time when the subject organism entered the identified state. In the illustrated example, the timing notification section 43 shows text "Time when current state was reached" and text "1 day ago," which is the time when the subject organism entered the identified state. The time when the subject organism entered the identified state is identified by the state identification unit 109 based on the relationship between the time point at which the relevant comparative organisms entered the overall state associated with the extracted statistical information and the time point at which the biomolecule satisfying the sameness/similarity condition was obtained. In this example, in the extracted statistical information shown in FIG. 7F, the time point at which the biomolecule satisfying the sameness/similarity condition, i.e., the biomolecule shown in the dashed-line portion A in the figure, was obtained is "Day 1," which is one of the individual states. Furthermore, the time point at which the relevant comparative organisms entered the overall state "influenza" corresponds to the time point at which the biomolecule associated with "infection," which is one of the individual states, was obtained, and this time point is one day before the time point of "Day 1." Therefore, the state identification unit 109 identifies the time when the subject organism entered the identified state as one day ago, which is thus displayed as "1 day ago" in the timing notification section 43.

The future state notification section 44 shows a future state of the subject organism identified by the state identification unit 109 along with the time when this future state will be reached. A future state of the subject organism identified by the state identification unit 109 may be referred to hereinafter as a "future state." In the illustrated example, the future state notification section 44 shows text "Future State," as well as text "1 day later: Fever" and text "5 days later: Normal temperature," respectively including a future state and the time when the future state will be reached. Note that the future state is one of the individual states associated with the extracted statistical information and is associated with a time point later than the time point at which the biomolecule satisfying the sameness/similarity condition was obtained. The time when the future state will be reached is identified by the state identification unit 109 based on the relationship between the time point at which the biomolecule satisfying the sameness/similarity condition was obtained and the time point with which the future state is associated. In this example, in the statistical information shown in FIG. 7F, the time points associated with the future states "fever" and "normal temperature" are "Day 2" and "Day 6," respectively, which are one day and five days after the time point at which the biomolecule satisfying the sameness/similarity condition was obtained. Therefore, the state identification unit 109 identifies the time when the subject organism will enter the future states "fever" and "normal temperature" as one day later and five days later, respectively, which are thus displayed as "1 day later" and "5 days later" in the future state notification section 44.

As described above, in the present embodiment, the detection unit 107 detects, from among a plurality of biomolecules shown in the subject distribution information, one or more biomolecules satisfying the change condition. The target determination unit 108 determines comparative statistical information according to each partitioned region including a corresponding biomolecule satisfying the change condition. The state identification unit 109 then identifies a state of the subject organism based on the relationship between the biomolecules satisfying the change condition and one or more biomolecules shown in the comparative statistical information. That is, in the present embodiment, the state identification unit 109 identifies a state of the subject organism according to each target associated with a biomolecule among a plurality of biomolecules, the target satisfying a condition defined for changes in biomolecules identified from molecular information. Examples of a condition defined for changes in biomolecules include the change condition. Examples of the target associated with a biomolecule include a biomolecule included in each partitioned region among a plurality of biomolecules. This feature allows a state of the subject organism to be identified using not only one biomolecule but also a plurality of biomolecules. Furthermore, based on the identified state of the subject organism, a method for improving the state of the subject organism may be determined. That is, the result of identifying the state of the subject organism may be used to determine how to deal with the subject organism.

In particular, the state identification system 1 according to the present embodiment creates a two-dimensional electrophoresis image using components collected from a subject organism without predefining which biomolecules are to be collected from the subject organism, and identifies a state of the subject organism based on biomolecules shown in the created two-dimensional electrophoresis image. Here, a method is also contemplated in which biomolecules to be collected from a subject organism are predefined according to the state of the subject organism to be identified, and a state of the subject organism is identified using only the predefined biomolecules. However, such a method is unable to identify a state of the subject organism based on other biomolecules different from the predefined biomolecules even when these other biomolecules exhibit changes over time. Accordingly, in the present embodiment, a state of a subject organism is identified using all biomolecules contained in components collected from the subject organism.

Note that the state identification unit 109 according to the present embodiment identifies not only a current state of the subject organism but also a future state of the subject organism (see FIG. 12). That is, the above phrase "identifying a state of the subject organism" includes not only identifying a current state of the subject organism but also identifying a future state of the subject organism.

In the present embodiment, biomolecules shown in the two-dimensional electrophoresis image include not only those whose functions are known but also those whose functions are unknown. Here, a method is also contemplated in which a state of the subject organism is identified using only biomolecules whose functions are known. However, such a method is unable to identify a state of the subject organism if none of the biomolecules whose functions are known satisfies the change condition. Accordingly, in the present embodiment, not only biomolecules whose functions are known but also biomolecules whose functions are unknown are used to identify a state of the subject organism.

In the present embodiment, the output unit 110 outputs information relating to a state of the subject organism, the information being derived from each target, associated with a corresponding biomolecule, satisfying the condition defined for changes in biomolecules identified from molecular information. This feature allows information on a state of the subject organism to be output using not only a single biomolecule but also a plurality of biomolecules.

In particular, in the present embodiment, a target associated with a biomolecule is a portion of the biomolecule, the portion satisfying the change condition. This feature allows a state of the subject organism to be identified even when the biomolecule as a whole does not satisfy the condition defined for changes in biomolecules.

In the present embodiment, for organism "A," the state of organism "A" is identified as being "influenza" in response to biomolecules included in Regions 14 and 15 and a biomolecule included in Region 20 each satisfying the change condition. Also, as described above, the state of organism "A" is identified as being "COVID-19" when a biomolecule included in Region 20 and a biomolecule included in Region 7 of organism "A" each satisfy the change condition. That is, in the present embodiment, the state identification unit 109 identifies a state of the subject organism depending on which targets, associated with respective biomolecules among a plurality of biomolecules, satisfy the change condition. This feature allows a state of the subject organism to be identified according to a plurality of biomolecules.

In the present embodiment, the distribution information includes information relating to a biomolecule in each of a plurality of segments that are defined according to physical quantities of biomolecules. Further, the change condition is defined for each of the plurality of segments. Examples of the physical quantities of biomolecules include their molecular weights and isoelectric points. Examples of the segments include partitioned regions. This feature allows a state of the subject organism to be identified according to physical quantities of targets associated with respective biomolecules that satisfy the change condition.

In the present embodiment, for organism "A," the state of organism "A" is identified as being "influenza" in response to a biomolecule included in Region 20 and biomolecules included in Regions 14 and 15 each satisfying the change condition. That is, in the present embodiment, the state identification unit 109 identifies a state of the subject organism in response to both a specific target associated with a first biomolecule and a specific target associated with a second biomolecule in the subject organism satisfying the change condition. This feature improves the accuracy of identifying a state of the subject organism, compared to an implementation where a state of the subject organism is identified only in response to a specific target associated with a single biomolecule satisfying the change condition.

In particular, in the present embodiment, for organism "A," a biomolecule in Region 20, which decreased in amount, and a biomolecule in Region 14, which increased in amount, both satisfy the change condition. That is, in the present embodiment, an increase in a physical quantity of a specific target associated with a first biomolecule satisfies the change condition, and a decrease in the physical quantity of a specific target associated with a second biomolecule also satisfies the change condition. This feature allows a state of the subject organism to be identified even when the trend of change in physical quantity of the first biomolecule differs from that of the second biomolecule.

Further, in the present embodiment, for organism "A," the state of organism "A" is identified as being "influenza" in response to a biomolecule included in Region 20 and a biomolecule included in Region 14 satisfying the change condition and a biomolecule included in Region 7 not satisfying the change condition. Also, for organism "A," the state of organism "A" is identified as being "COVID-19" when a biomolecule included in Region 20 and a biomolecule included in Region 7 satisfy the change condition and a biomolecule included in Region 14 does not satisfy the change condition. That is, in the present embodiment, the state identification unit 109 identifies a state of the subject organism as being a first state when a specific target associated with a first biomolecule and a specific target associated with a second biomolecule in the subject organism satisfy the change condition and a specific target associated with a third biomolecule does not satisfy the change condition. Further, the state identification unit 109 identifies a state of the subject organism as being a second state when the specific target associated with the first biomolecule and the specific target associated with the third biomolecule in the subject organism satisfy the change condition and the specific target associated with the second biomolecule does not satisfy the change condition. This feature allows a state of the subject organism to be identified even when a common biomolecule satisfies the condition defined for changes in biomolecules in both the first state and the second state.

In the present embodiment, for organism "A," the state of organism "A" is identified as being "influenza" in response to both a portion included in Region 14 of a biomolecule and a portion included in Region 15 of that biomolecule satisfying the change condition. That is, in the present embodiment, the state identification unit 109 identifies a state of the subject organism in response to both a first portion and a second portion of a specific biomolecule satisfying the change condition. This feature improves the accuracy of identifying a state of the subject organism, compared to an implementation where a state of the subject organism is identified only in response to a single portion of a biomolecule satisfying the change condition.

In particular, in the present embodiment, for organism "A," the state of organism "A" is identified as being "influenza" in response to an increase in amount of a portion of a biomolecule included in Region 14 satisfying the change condition and a decrease in amount of another portion of that biomolecule included in Region 15 satisfying the change condition. That is, in the present embodiment, an increase in a specific physical quantity of a first portion satisfies the change condition, and a decrease in the specific physical quantity of a second portion satisfies the change condition. This feature allows a state of the subject organism to be identified even when the trend of change in physical quantity of the first portion differs from that of the second portion.

It should be noted that the method for identifying a state by the state identification unit 109 is not limited to the above example. For example, based on the relationship with corresponding biomolecules shown in the comparative statistical information, a state identified for the subject organism may vary depending on whether, for a biomolecule consisting of Regions 1 to 5, a change in amount of a portion included in Region 1 or a change in amount of a portion included in Region 5 satisfies the change condition. That is, the state identification unit 109 may identify a state of the subject organism as being a first state when a first portion of a specific biomolecule of the subject organism satisfies the change condition and a second portion of the biomolecule does not satisfy the change condition, and may identify a state of the subject organism as being a second state, different from the first state, when the first portion does not satisfy the change condition and the second portion satisfies the change condition. A state of a biomolecule may vary depending on a site where the biomolecule is post-translationally modified. For example, when a phosphate group is added to a specific biomolecule containing amino acids, the activation state and function of the specific biomolecule vary between a case where the 20th amino acid is phosphorylated and a case where the 45th amino acid is phosphorylated. In the present embodiment, respective states of the subject organism can be identified based on such different states of a biomolecule. More specifically, respective states of the subject organism can be identified based on changes in physical quantity of the biomolecule in different states.

### (Another Example of State Identification Process)

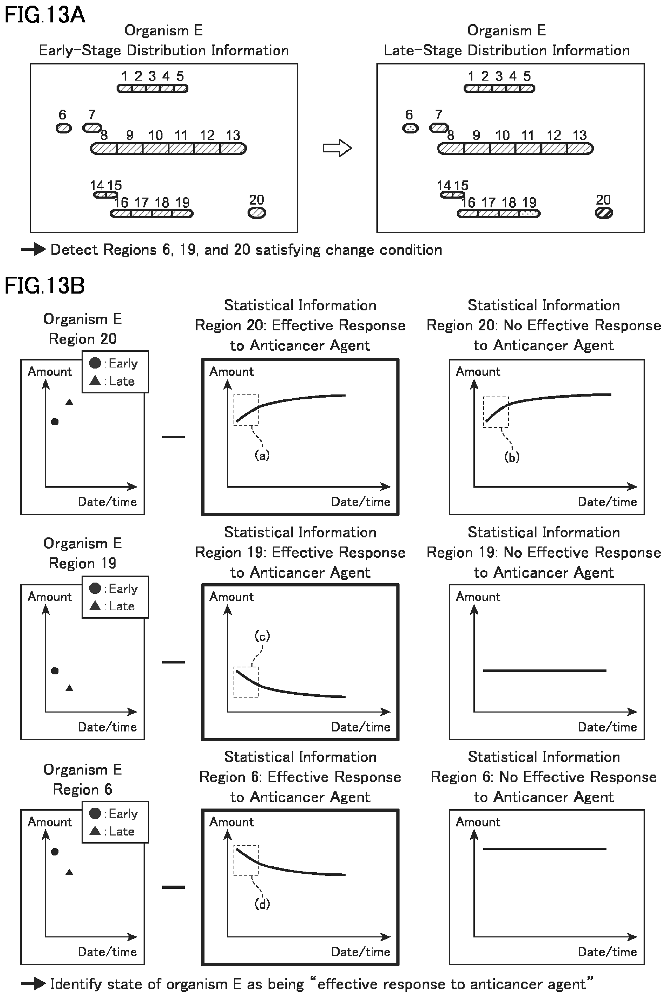

The following is a description of an example of the state identification process performed when subject distribution information different from the example shown in FIG. 9A is used. FIGS. 13A to 14C illustrate an example sequence until a state of a subject organism is identified in the state identification process.

The following describes a case where the subject organism is organism "E" and the used distribution information includes two sets of subject distribution information shown in FIG. 13A. The used distribution information shown in FIG. 13A includes early-stage distribution information and late-stage distribution information for organism "E". The early-stage distribution information and the late-stage distribution information each show a distribution of seven mutually different biomolecules, as in the distribution information shown in FIG. 6A. The early-stage distribution information shown in FIG. 13A is subject distribution information showing biomolecules contained in components obtained from organism "E" on the day before the administration of an anticancer agent to organism "E." The late-stage distribution information shown in FIG. 13A is subject distribution information showing biomolecules contained in components obtained from organism "E" on the day after the administration of the anticancer agent to organism "E." Organism "E" is, for example, a human. The region recognition unit 103 recognizes one or more regions where biomolecules are present in each set of the used distribution information. In each set of the used distribution information, the partitioning unit 104 partitions the one or more regions where biomolecules are present into twenty regions consisting of Regions 1 to 20, in the same manner as the partitioning performed on the comparative distribution information (see FIG. 6B).

For each set of the used distribution information, the amount determination unit 105 determines the amount of biomolecule included in each partitioned region. The detection unit 107 determines, for each partitioned region, whether a biomolecule satisfies the change condition, thereby detecting one or more biomolecules determined to satisfy the change condition. In this example, in each of Regions 6, 9, and 20 shown in FIG. 13A, the amount of biomolecule shown in the late-stage distribution information has changed from that shown in the early-stage distribution information. More specifically, in each of Regions 6 and 19, the amount of biomolecule shown in the late-stage distribution information is less than that shown in the early-stage distribution information. In Region 20, the amount of biomolecule shown in the late-stage distribution information is greater than that shown in the early-stage distribution information. As such, the biomolecules in Regions 6, 19, and 20 shown in the early-stage distribution information and the late-stage distribution information are detected by the detection unit 107 as satisfying the change condition.

Next, the target determination unit 108 determines comparative statistical information to be compared with the biomolecules satisfying the change condition, i.e., the detected biomolecules. In this example, as shown in FIG. 13B, the target determination unit 108 determines each set of statistical information for Region 20 as the comparative statistical information corresponding to the detected biomolecule shown in Region 20 for organism "E". Also, the target determination unit 108 determines each set of statistical information for Region 19 as the comparative statistical information corresponding to the detected biomolecule shown in Region 19 for organism "E," and determines each set of statistical information for Region 6 as the comparative statistical information corresponding to the detected biomolecule shown in Region 6 for organism "E." As shown in FIG. 13B, these sets of comparative statistical information include statistical information associated with an overall state "effective response to anticancer agent" and statistical information associated with an overall state "no effective response to anticancer agent." FIG. 13B illustrates information to be compared by the state identification unit 109. More specifically, FIG. 13B illustrates the biomolecules contained in the early-stage distribution information and the late-stage distribution information, comparative statistical information associated with an overall state "no effective response to anticancer agent," and comparative statistical information associated with an overall state "no effective response to anticancer agent." In FIG. 13B, the symbols of a black circle each indicate the biomolecule shown in the early-stage distribution information, and the symbols of a black triangle each indicate the biomolecule shown in the late-stage distribution information.

Next, the state identification unit 109 extracts, from the comparative statistical information, one or more biomolecules satisfying the sameness/similarity condition. In this example, in the comparative statistical information for Region 20 associated with "effective response to anticancer agent," the dashed-line portion (a) in the figure indicates an increase in biomolecule amount over time. Thus, the state identification unit 109 determines that the biomolecule shown in this portion (a) satisfies the sameness/similarity condition with respect to the detected biomolecule included in Region 20 for organism "E". Also, in the comparative statistical information for Region 20 associated with "no effective response to anticancer agent," the dashed-line portion (b) in the figure indicates an increase in biomolecule amount over time. Thus, the state identification unit 109 determines that the biomolecule shown in this portion (b) satisfies the sameness/similarity condition with respect to the detected biomolecule included in Region 20 for organism "E". In this case, the state identification unit 109 compares one or more biomolecules shown in the extracted comparative statistical information with the detected biomolecules shown in another partitioned region for which comparison has not yet been performed. More specifically, the state identification unit 109 compares the detected biomolecule included in Region 19 for organism "E" with the comparative statistical information for Region 19. Also, the state identification unit 109 compares the detected biomolecule included in Region 6 for organism "E" with the comparative statistical information for Region 6.

In the comparative statistical information for Region 19 associated with "effective response to anticancer agent," the dashed-line portion (c) in the figure indicates a decrease in biomolecule amount over time. Thus, the state identification unit 109 determines that the biomolecule shown in this portion (c) satisfies the sameness/similarity condition with respect to the detected biomolecule included in Region 19 for organism "E". Also, in the comparative statistical information for Region 6 associated with "effective response to anticancer agent," the dashed-line portion (d) in the figure indicates a decrease in biomolecule amount over time. Thus, the state identification unit 109 determines that the biomolecule shown in this portion (d) satisfies the sameness/similarity condition with respect to the detected biomolecule included in Region 6 for organism "E". On the other hand, the comparative statistical information for Region 19 associated with "no effective response to anticancer agent" indicates no change in biomolecule amount over time. Thus, the state identification unit 109 determines that this biomolecule does not satisfy the sameness/similarity condition with respect to the detected biomolecule shown in Region 19 for organism "E". Also, the comparative statistical information for Region 6 associated with "no effective response to anticancer agent" indicates no change in biomolecule amount over time. Thus, the state identification unit 109 determines that this biomolecule does not to satisfy the sameness/similarity condition with respect to the detected biomolecule shown in Region 6 for organism "E".

In this case, the state identification unit 109 extracts the biomolecules shown in the comparative statistical information associated with "effective response to anticancer agent" as biomolecules that satisfy the sameness/similarity condition. Also, the state identification unit 109 identifies organism "E" as being in the state of "effective response to anticancer agent," which is an overall state associated with the extracted comparative statistical information. Furthermore, the output unit 110 causes a state notification screen 40 (see FIG. 12) relating to the state identified by the state identification unit 109 to be displayed on the display 31 of the terminal 30.

In this example, both the biomolecule shown in the comparative statistical information associated with "effective response to anticancer agent" and the biomolecule shown in the comparative statistical information associated with "no effective response to anticancer agent" satisfy the sameness/similarity condition with respect to the detected biomolecule included in Region 20.

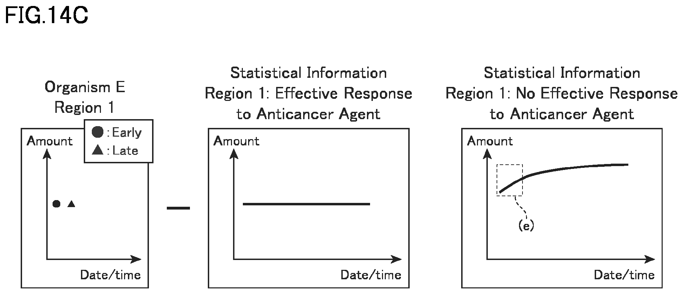

FIG. 14C illustrates a biomolecule included in Region 1 across the early-stage distribution information and the late-stage distribution information, comparative statistical information for Region 1 associated with "effective response to anticancer agent," and comparative statistical information for Region 1 associated with "no effective response to anticancer agent." In each of the diagrams shown in FIG. 14C, the horizontal axis represents the time point at which the biomolecule was obtained, and the vertical axis represents the amount of biomolecule. In FIG. 14C, the symbol of a black circle indicates the biomolecule shown in the early-stage distribution information, and the symbol of a black triangle indicates the biomolecule shown in the late-stage distribution information. As shown in FIG. 14C, in Region 1 for organism "E," there is no change in biomolecule amount between the early-stage distribution information and the late-stage distribution information. There is also no change in biomolecule amount over time in the comparative statistical information for Region 1 associated with "effective response to anticancer agent." On the other hand, in the comparative statistical information for Region 1 associated with "no effective response to anticancer agent," the dashed-line portion (e) in the figure indicates an increase in biomolecule amount over time. Therefore, when the biomolecule of organism "E" included in Region 20 exhibits a change like at the portion (b) in FIG. 13B and the biomolecule of organism "E" included in Region 1 exhibits a change like that at the portion (e) in FIG. 14C, the state identification unit 109 identifies a state of organism "E" as being "no effective response to anticancer agent."

As described above, in the present embodiment, the information acquisition unit 101 acquires the used distribution information that enables the identification of a state of biomolecules over time, from before a specific action acts on the subject organism to after the specific action has acted thereon. Examples of the specific action include administration of an anticancer agent. Then, the state identification unit 109 identifies a state of the subject organism as being a first state related to the specific action when a specific target associated with a specific biomolecule satisfies the change condition, and identifies a state of the subject organism as being a second state related to the specific action when the specific target associated with the specific biomolecule does not satisfy the change condition. Examples of the specific target associated with the specific biomolecule include a portion of the biomolecule shown in Region 19 (see FIGS. 13A and 13B). Examples of the first state include a state in which the subject organism exhibits an effect resulting from administration of an anticancer agent. Examples of the second state include a state in which the subject organism exhibits no effect resulting from administration of the anticancer agent. This feature enables the identification of an effect on the subject organism resulting from a specific action acting thereon.

In particular, in the present embodiment, the state identification unit 109 identifies a state of the subject organism as being a first state when a specific target associated with a first biomolecule satisfies the change condition and a specific target associated with a second biomolecule does not satisfy the change condition, and identifies a state of the subject organism as being a second state when the specific target associated with the first biomolecule does not satisfy the change condition and the specific target associated with the second biomolecule satisfies the change condition. Examples of the specific target associated with the second biomolecule include a portion of the biomolecule shown in Region 1 (see FIGS. 13A and 14C). This feature can improve the accuracy of identifying a state of the subject organism as being the second state, compared to an implementation in which a state of the subject organism is identified as being the second state only in response to a specific target associated with the first biomolecule not satisfying the change condition.

In the present embodiment, when the biomolecule included in Region 20, the biomolecule included in Region 19, and the biomolecule included in Region 6 all satisfy the change condition in the used distribution information, the state identification unit 109 identifies a state of the subject organism as being the first state; however, this is not limiting. For example, when the biomolecule included in Region 19 satisfies the change condition and the biomolecule included in Region 6 does not satisfy the change condition in the used distribution information, in response to a biomolecule shown in comparative statistical information for Region 19 associated with "effective response to anticancer agent" satisfying the sameness/similarity condition, the state identification unit 109 may identify a state of the subject organism as being "effective response to anticancer agent." That is, the state identification unit 109 identifies a state of the subject organism as being a first state when both a specific target associated with a first biomolecule and a specific target associated with a second biomolecule satisfy the change condition, and may also identify a state of the subject organism as being the first state when the specific target associated with the first biomolecule does not satisfy the change condition and the specific target associated with the second biomolecule satisfies the change condition. Examples of the specific target associated with the first biomolecule include a portion of the biomolecule included in Region 19. Examples of the specific target associated with the second biomolecule include a portion of the biomolecule included in Region 6 (see FIGS. 13A and 13B). This feature helps prevent the subject organism from being mistakenly identified as being in a state different from the first state, despite the subject organism being in the first state.

In the present embodiment, the state identification unit 109 identifies, as a state of the subject organism related to a specific action, an overall state that is associated with one or more individual health states; however, this is not limiting. For example, the state identification unit 109 may identify, as a state of the subject organism, an overall state that is associated with one or more individual physical states, based on changes in biomolecules of the subject organism. For example, the state identification unit 109 may identify a state of the subject organism as being "training effect present," which is an overall state associated with a physical state "muscle soreness" (see FIG. 4A). In another example, the state identification unit 109 may identify a state of the subject organism as being "training effect absent," which is an overall state associated with a physical state "fatigue." The overall states "training effect present" and "training effect absent" are examples of the state relating to training performed by the subject organism. Performing the training is an example of the specific action. Still alternatively, for example, the state identification unit 109 may identify, as a state of the subject organism, an overall state that is associated with one or more individual brain states, based on changes in biomolecules of the subject organism. For example, the state identification unit 109 may identify a state of the subject organism as being "improved memory," which is an overall state associated with a brain state "3 hours of learning" (see FIG. 4A) and a physical state "8 hours of sleep". In another example, the state identification unit 109 may identify a state of the subject organism as being "reduced memory," which is an overall state associated with a brain state "3 hours of learning" and a physical state "4 hours of sleep". In this manner, two or more pieces of individual state information may be associated with overall state information. The overall states "improved memory" and "reduced memory" are examples of the state relating to learning by the subject organism. Learning is an example of the specific action. The state of the subject organism to be identified by the state identification unit 109 after a specific action has acted thereon may be not only a state in which the specific action is effective or ineffective, but also a state in which a positive or negative effect is produced by the specific action. Still alternatively, for example, the state identification unit 109 may identify, as a state of the subject organism, an overall state that is associated with one or more individual cosmetic states, based on changes in biomolecules of the subject organism. For example, the state identification unit 109 may identify a state of the subject organism as being "skin age: 40," which is an overall state associated with a cosmetic state "skin cream A used" (see FIG. 4A). In another example, the state identification unit 109 may identify a state of the subject organism as being "skin age: 25," which is an overall state associated with a cosmetic state "skin cream B used." The overall states "skin age: 40" and "skin age: 25" are examples of the state relating to the use of skin cream by the subject organism. The use of skin cream is an example of the specific action.

### (Another Example of State Identification Process)

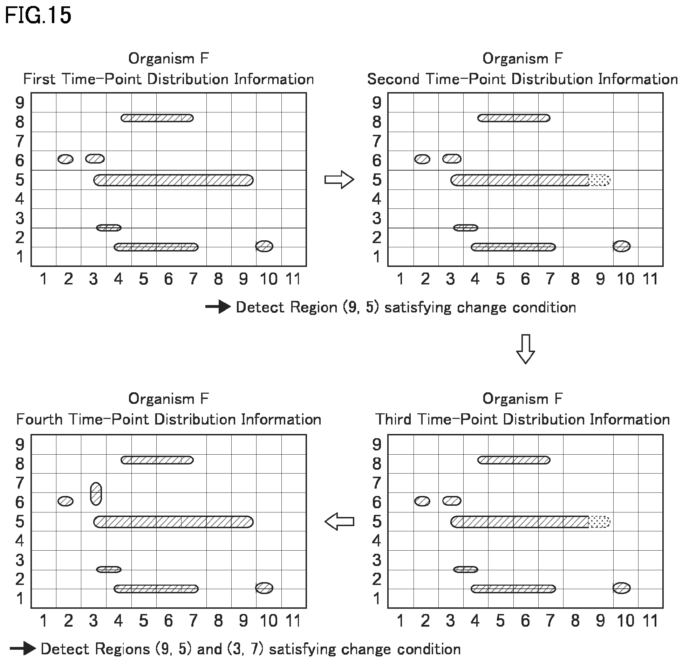

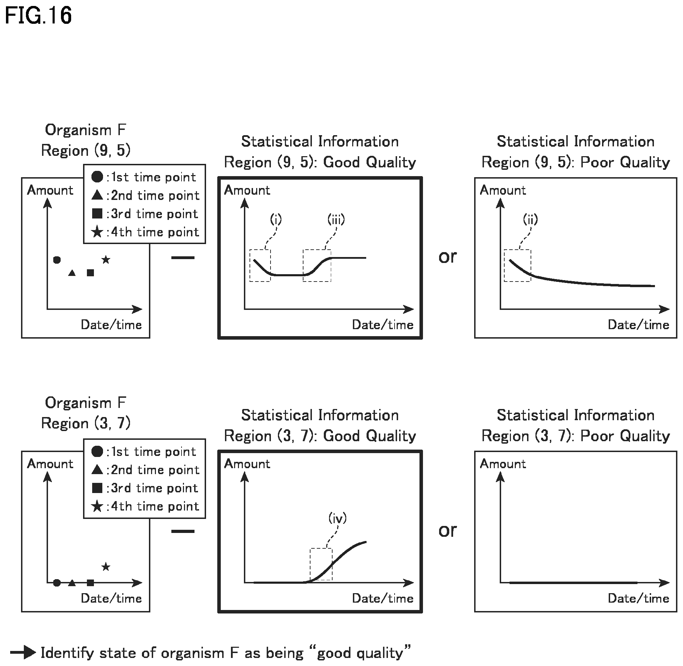

The following is a description of an example of the state identification process performed when subject distribution information different from the examples shown in FIGS. 9A and 13A is used. FIGS. 15 and 16 illustrate an example sequence until a state of a subject organism is identified in the state identification process.

The following describes a case where the subject organism is organism "F" and the used distribution information includes four sets of subject distribution information shown in FIG. 15. FIG. 15 illustrates four sets of subject distribution information as the used distribution information for organism "F," each corresponding to a different time point at which biomolecules of organism "F" were obtained. These four sets of used distribution information each show a distribution of seven mutually different biomolecules, as in the distribution information shown in FIG. 6A. Hereinafter, the four sets of used distribution information shown in FIG. 15 may be referred to as "first time-point distribution information," "second time-point distribution information," "third time-point distribution information," and "fourth time-point distribution information" in order from the distribution information with the earliest time point at which biomolecules were obtained. Organism "F" is, for example, banana.

In this example, the partitioning unit 104 partitions the entire region in each set of used distribution information. To further illustrate, each set of used distribution information is, as shown in FIG. 15, a two-dimensional Cartesian coordinate system in which the horizontal axis represents coordinate values from 1 to 11 corresponding to isoelectric points of biomolecules and the vertical axis represents coordinate values from 1 to 9 corresponding to molecular weights of biomolecules. A larger coordinate value on the horizontal axis indicates a larger isoelectric point of a biomolecule, and a larger coordinate value on the vertical axis indicates a larger molecular weight of a biomolecule. Thus, the partitioning unit 104 partitions the region shown in each set of used distribution information into regions corresponding to respective coordinates. In the example shown in FIG. 15, the entire region is partitioned in each set of used distribution information, resulting in partitioned regions where biomolecules are present and partitioned regions where no biomolecules are present.

Although not shown in the figures, in this example, the partitioning unit 104 also partitions the entire region in each comparative distribution information stored in the storage unit 102, in the same manner as the partitioning performed on the used distribution information shown in FIG. 15. Also, the amount determination unit 105 determines, for each partitioned region, the amount of biomolecule included in that partitioned region in each comparative distribution information. Furthermore, the statistical processing unit 106 calculates statistics of the amount of biomolecule for each partitioned region and creates statistical information for each partitioned region.

For each set of used distribution information, the amount determination unit 105 determines the amount of biomolecule included in each partitioned region. The detection unit 107 determines, for each partitioned region, whether a biomolecule satisfies the change condition, thereby detecting one or more biomolecules determined to satisfy the change condition. In this example, in the region where a horizontal coordinate value is "9" and a vertical coordinate value is "5," that is, in Region (9, 5), the amount of biomolecule shown in the second time-point distribution information is smaller than that shown in the first time-point distribution information. In Region (9, 5), the amount of biomolecule shown in the fourth time-point distribution information is greater than that shown in the third time-point distribution information. Furthermore, in Region (3, 7), while no biomolecule is shown in the third time-point distribution information, a biomolecule is shown in the fourth time-point distribution information. In this case, the detection unit 107 detects the biomolecule shown in Region (9, 5) in the first time-point distribution information and the second time-point distribution information as satisfying the change condition. Also, the detection unit 107 detects the biomolecule shown in Region (9, 5) in the third time-point distribution information and the fourth time-point distribution information as satisfying the change condition. Furthermore, the detection unit 107 detects the biomolecule shown in Region (3, 7) in the fourth time-point distribution information as satisfying the change condition. In Region (3, 7), there is no change in biomolecule amount between the first, second, and third time-point distribution information. More specifically, in Region (3, 7), no biomolecule is shown in the first, second, and third time-point distribution information.

Next, the target determination unit 108 determines comparative statistical information to be compared with the biomolecules satisfying the change condition, i.e., the detected biomolecules. In this example, as shown in FIG. 16, the target determination unit 108 determines each set of statistical information for Region (9, 5) as comparative statistical information corresponding to the detected biomolecule shown in Region (9, 5) for organism "F". Also, the target determination unit 108 determines each set of statistical information for Region (3, 7) as comparative statistical information corresponding to the detected biomolecule shown in Region (3, 7) for organism "F." As shown in FIG. 16, these sets of comparative statistical information include statistical information associated with an overall state "good quality" and statistical information associated with an overall state "poor quality." FIG. 16 illustrates information to be compared by the state identification unit 109. More specifically, FIG. 16 illustrates the biomolecules shown in the respective sets of used distribution information, comparative statistical information associated with an overall state "good quality," and comparative statistical information associated with an overall state "poor quality."

For the biomolecules of organism "F" shown in FIG. 16, the symbols of a black circle each indicate the corresponding biomolecule shown in the first time-point distribution information, the symbols of a black triangle each indicate the corresponding biomolecule shown in the second time-point distribution information, the symbols of a black square each indicate the corresponding biomolecule shown in the third time-point distribution information, and the symbols of a black star each indicate the corresponding biomolecule shown in the fourth time-point distribution information.

Next, the state identification unit 109 extracts, from the comparative statistical information, one or more biomolecules satisfying the sameness/similarity condition. In this example, in the comparative statistical information for Region (9, 5) associated with an overall state "good quality," the dashed-line portion (i) in the figure indicates a decrease in biomolecule amount the over time. Thus, the state identification unit 109 determines that the biomolecule shown in this portion (i) satisfies the sameness/similarity condition with respect to the biomolecule shown in Region (9, 5) in the first time-point distribution information and the second time-point distribution information. Also, in the comparative statistical information for Region (9, 5) associated with an overall state "poor quality," the dashed-line portion (ii) in the figure indicates a decrease in biomolecule amount over time. Thus, the state identification unit 109 determines that the biomolecule shown in this portion (ii) satisfies the sameness/similarity condition with respect to the biomolecule shown in Region (9, 5) in the first time-point distribution information and the second time-point distribution information.

In this example, in the comparative statistical information for Region (9, 5) associated with an overall state "good quality," the dashed-line portion (iii) in the figure indicates an increase in biomolecule amount over time. Thus, the state identification unit 109 determines that the biomolecule shown in this portion (iii) satisfies the sameness/similarity condition with respect to the biomolecule shown in Region (9, 5) in the third time-point distribution information and the fourth time-point distribution information. Also, in the comparative statistical information for Region (3, 7) associated with an overall state "good quality," the dashed-line portion (iv) in the figure indicates an increase in biomolecule amount over time. Thus, the state identification unit 109 determines that the biomolecule shown in this portion (iv) satisfies the sameness/similarity condition with respect to the biomolecule shown in Region (3, 7) in the fourth time-point distribution information. On the other hand, the comparative statistical information for Region (9, 5) associated with an overall state "poor quality" indicates no increase in biomolecule amount over time. In this case, the state identification unit 109 determines that the biomolecule shown in this comparative statistical information does not satisfy the sameness/similarity condition with respect to the biomolecule shown in Region (9, 5) in the third time-point distribution information and the fourth time-point distribution information. Also, the comparative statistical information for Region (3, 7) associated with an overall state "poor quality" indicates no change in biomolecule amount over time. In this case, the state identification unit 109 determines that the biomolecule shown in this comparative statistical information does not satisfy the sameness/similarity condition with respect to the biomolecule shown in Region (3, 7) in the fourth time-point distribution information.

In this case, the state identification unit 109 extracts the biomolecules shown in the comparative statistical information associated with "good quality" as biomolecules satisfying the sameness/similarity condition. Also, the state identification unit 109 identifies organism "F" as being in the state of "good quality," which is an overall state associated with the extracted comparative statistical information. Furthermore, the output unit 110 causes a state notification screen 40 (see FIG. 12) relating to the state identified by the state identification unit 109 to be displayed on the display 31 of the terminal 30.

As described above, no biomolecule is shown in Region (3, 7) in the first, second, and third time-point distribution information included in the four sets of used distribution information shown in FIG. 15. That is, in the present embodiment, the used distribution information includes information relating to the presence of a biomolecule at a plurality of time points in each of a plurality of partitioned regions, and the plurality of partitioned regions include a region where no biomolecule of the subject organism is present at at least one of the plurality of time points. This feature allows a state of the subject organism to be identified based on a change in a biomolecule in a region where no biomolecule had been present.

Furthermore, in the present embodiment, the information acquisition unit 101 acquires information including information that enables the identification of a change in one or more biomolecules of the subject organism during a first period and information that enables the identification of a change in one or more biomolecules during a second period subsequent to the first period. Examples of the first period include a period from the time point at which the biomolecules shown in the first time-point distribution information were obtained to the time point at which the biomolecules shown in the second time-point distribution information were obtained. Examples of the second period include a period from the time point at which the biomolecules shown in the third time-point distribution information were obtained to the time point at which the biomolecules shown in the fourth time-point distribution information were obtained. Then, the state identification unit 109 identifies a state of the subject organism based on one or more targets, associated with respective biomolecules, that satisfy the change condition for a change during the first period and based on one or more targets, associated with respective biomolecules, that satisfies the change condition for a change during the second period. This feature can improve the accuracy of identifying a state of the subject organism, compared to an implementation in which a state of the subject organism is identified only in response to a biomolecule satisfying the change condition for a change during a single period.

In particular, in the present embodiment, a target associated with a biomolecule satisfying the change condition for a change during the first period is the same as a target associated with a biomolecule satisfying the change condition for a change during the second period. Examples of the target associated with a biomolecule include the biomolecule included in Region (9, 5) (see FIGS. 15 and 16). This feature allows a state of the subject organism to be identified based on changes in a specific biomolecule over a plurality of periods.

### (SECOND EMBODIMENT)

A second embodiment of the present disclosure will now be described. The second embodiment is similar to the first embodiment in that the state identification system 1 identifies a state of a subject organism. On the other hand, the second embodiment differs from the first embodiment in that a user of the state identification system 1 can select the type of state to be identified by the state identification unit 109 for the subject organism. The second embodiment will be described below. The configuration of the second embodiment is the same as that of the first embodiment, except for the differences described below. In the following, only the differences from the first embodiment will be described, and redundant descriptions of identical features and components may be omitted.

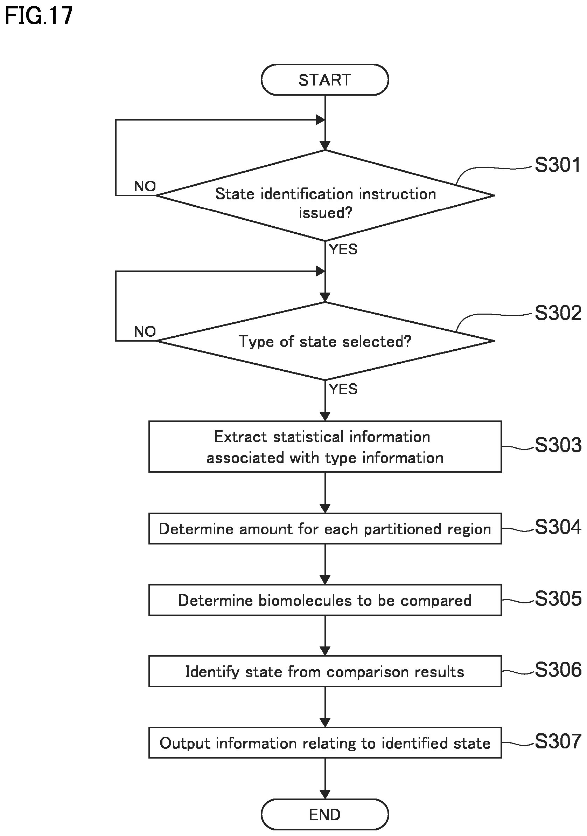

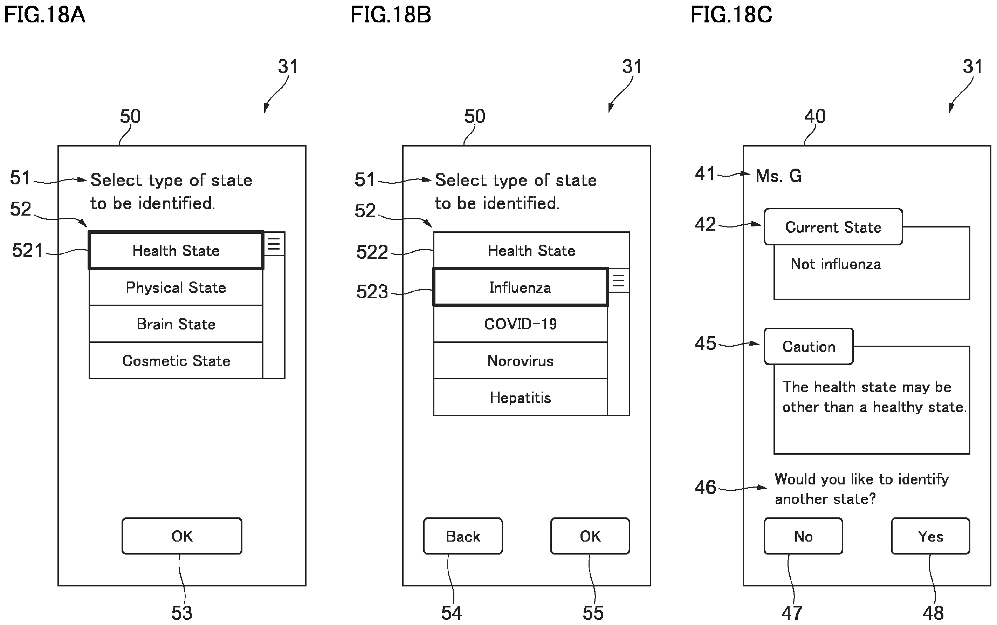

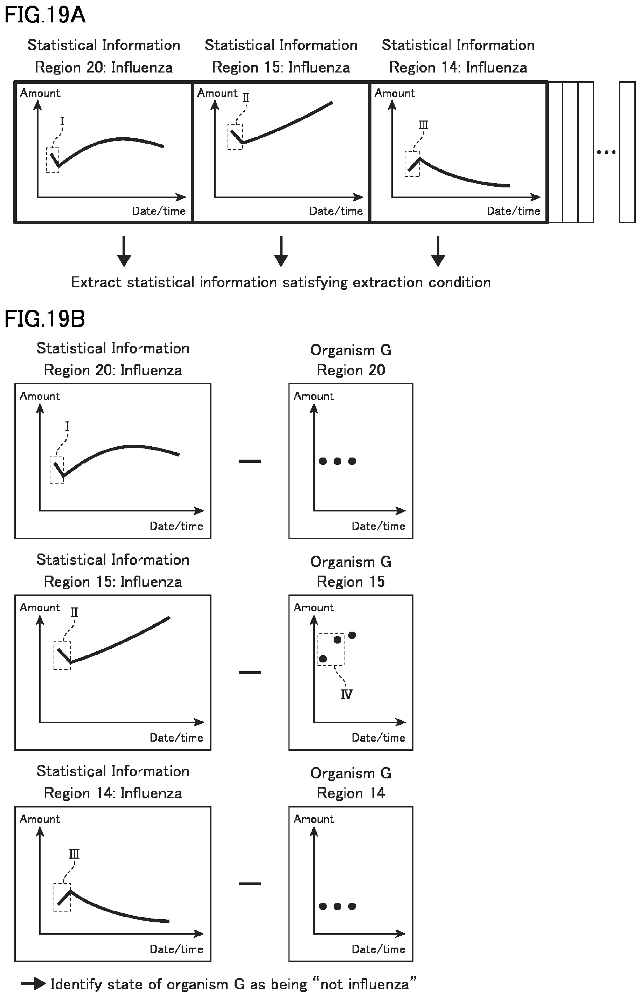

FIG. 17 is a flowchart illustrating a state identification process according to the second embodiment. FIGS. 18A to 18C illustrate images displayed on the display 31 of the terminal 30 in cases where the state identification process according to the second embodiment is performed. FIGS. 19A and 19B illustrate an example sequence until a state of a subject organism is identified in the state identification process according to the second embodiment. The following describes a case where the subject organism is organism "G," which is, for example, a human. In the present embodiment as well, the state identification process is initiated in response to a user of the state identification system 1 specifying subject distribution information used for the state identification process and instructing the server device 10 to execute the state identification process.

First, the information acquisition unit 101 determines whether an instruction to identify a state of the subject organism has been issued (S301 in FIG. 17). In response to the user inputting an instruction to identify a state of the subject organism to the terminal 30, a notification indicating the issuance of the instruction is transmitted from the terminal 30 to the server device 10. Based on receipt of the notification, the information acquisition unit 101 determines whether an instruction to identify a state of the subject organism has been issued. While the determination is negative, the information acquisition unit 101 repeats step 301.

Once the notification indicating the issuance of an instruction to identify a state of the subject organism is transmitted from the terminal 30 to the server device 10 (YES in S301), the information acquisition unit 101 determines whether the type of state to be identified by the state identification unit 109 has been selected (S302). In response to the user selecting, on the terminal 30, the type of state to be identified by the state identification unit 109, type information indicating the selected type of state is transmitted from the terminal 30 to the server device 10. Based on receipt of the type information, the information acquisition unit 101 determines whether the type of state to be identified by the state identification unit 109 has been selected. While the determination is negative, the information acquisition unit 101 repeats step 302.

In the state identification process in this example, in response to the user inputting an instruction to identify a state of the subject organism to the terminal 30, a selection screen 50 is displayed on the display 31 of the terminal 30, as shown in FIG. 18A. The selection screen 50 is a screen for allowing the user to select the type of state to be identified by the state identification unit 109. The selection screen 50 includes a selection prompting section 51, an item section 52, and an "OK" section 53. The selection prompting section 51 shows information for prompting the user to select the type of state to be identified by the state identification unit 109. In the illustrated example, the selection prompting section 51 shows text "Select type of state to be identified."

The item section 52 shows selectable items for the user. The item section 52 includes an item selection section 521. The item selection section 521 shows items categorized corresponding to respective types of states to be identified by the state identification unit 109. In the illustrated example, the item selection section 521 shows items including "health state," "physical state," "brain state," and "cosmetic state," which are categorized corresponding to respective states to be identified by the state identification unit 109. In the illustrated example, the item "health state" is selected in the item selection section 521.

In response to the user selecting the "OK" section 53 after selecting the item "health state" in the item selection section 521, a selected item section 522 and a detailed selection section 523 are displayed in the item section 52 of the selection screen 50 (see FIG. 18B). Also, the selection screen 50 displays a "Back" section 54 and an "OK" section 55.

The selected item section 522 of the item section 52 shows the item selected in the item selection section 521. In the illustrated example, the item "health state" is shown in the selected item section 522. The detailed selection section 523 of the item section 52 shows items further categorized based on the item shown in the selected item section 522. Items shown in the detailed selection section 523 are selectable by the user. In the illustrated example, the detailed selection section 523 shows items including "influenza," "COVID-19," "norovirus," and "hepatitis," which are the items further categorized based on the item "health state" shown in the selected item section 522. In the illustrated example, the item "influenza" is selected in the detailed selection section 523.

In response to the user selecting the "Back" section 54, the selection screen 50 shown in FIG. 18A is displayed again on the display 31 of the terminal 30. In response to the user selecting the "OK" section 55 after selecting the item "influenza" in the detailed selection section 523, type information indicating the selected type of state "influenza" is transmitted from the terminal 30 to the server device 10.

Once the information acquisition unit 101 acquires the type information (YES in S302), the process proceeds to the next step. The target determination unit 108 extracts, from statistical information stored in the storage unit 102, statistical information with which the type information acquired by the information acquisition unit 101 is associated as overall state information, and uses the extracted statistical information as statistical information to be compared with biomolecules of the subject organism (S303). More specifically, the target determination unit 108 extracts statistical information that is associated with the type information and relates to one or more regions in which biomolecules satisfying an extraction condition are shown. The extraction condition is a condition defined for the target determination unit 108 to extract statistical information to be compared with biomolecules of the subject organism. In the present embodiment, the extraction condition is defined from the viewpoint of extracting statistical information relating to one or more regions each showing a biomolecule whose amount has changed over time. In the present embodiment, the extraction condition is defined such that the amount of biomolecule included in the statistical information has changed by a predetermined value or more over time.

FIG. 19A illustrates example statistical information stored in the storage unit 102. In this example, as shown in FIG. 19A, the storage unit 102 stores statistical information with which the type information "influenza" is associated as overall state information. In the statistical information for Region 20 associated with "influenza", the dashed-line portion I in the figure indicates a decrease in biomolecule amount over time. Also, in the statistical information for Region 15 associated with "influenza", the dashed-line portion II in the figure indicates a decrease in biomolecule amount over time. Furthermore, in the statistical information for Region 14 associated with "influenza", the dashed-line portion III in the figure indicates an increase in biomolecule amount over time. In this case, the target determination unit 108 extracts, as statistical information satisfying the extraction condition, statistical information for Region 20, statistical information for Region 15, and statistical information for Region 14, each being associated with "influenza".

Next, for each set of used distribution information, the amount determination unit 105 determines the amount of biomolecule included in each partitioned region (S304). Then, the target determination unit 108 determines one or more biomolecules of the subject organism to be compared with those shown in the statistical information extracted in step S303 (S305). More specifically, the target determination unit 108 determines, as biomolecules to be compared, those included in partitioned regions having the same coordinates as those of the statistical information extracted in step S303.

In this example, statistical information for Region 20, statistical information for Region 15, and statistical information for 14 are extracted, as described above. In this case, the target determination unit 108 determines, as biomolecules to be compared, biomolecules included in Regions 20, 15, and 14 among those shown in the used distribution information for organism "G." FIG. 19B illustrates the statistical information extracted in step S303 and the biomolecules of the subject organism determined by the target determination unit 108 for comparison therewith. In FIG. 19B, statistical information for Region 20, statistical information for Region 15, and statistical information for 14 are shown, each being associated with "influenza." Also shown in FIG. 19B are the biomolecules of organism "G" in Regions 20, 15, and 14 in each set of used distribution information. In each of the diagrams shown in FIG. 19B, the horizontal axis represents the time point at which the biomolecules was obtained and the vertical axis represents the amount of biomolecule.

Next, the state identification unit 109 compares the statistical information with the biomolecules of the subject organism to identify a state of the subject organism based on the comparison results (S306). More specifically, the state identification unit 109 identifies a state of the subject organism based on whether one or more biomolecules determined by the target determination unit 108 for comparison satisfy a specific condition. The specific condition is a condition defined for the state identification unit 109 to identify a state of the subject organism as being the overall state associated with the statistical information. In the present embodiment, the specific condition is defined such that a difference between a change over time in the amount of a biomolecule of the subject organism and a change over time in the amount of a biomolecule satisfying the extraction condition in the statistical information is less than or equal to a predetermined value.

If any biomolecule of the subject organism satisfies the specific condition, the state identification unit 109 identifies a state of the subject organism as being the overall state associated with the statistical information extracted in step S303. If no biomolecule of the subject organism satisfies the specific condition, the state identification unit 109 identifies a state of the subject organism as being a state that is not the overall state associated with the statistical information extracted in Step S303. Note that the biomolecules of the subject organism determined by the target determination unit 108 for comparison may be biomolecules included in a plurality of partitioned regions. In this case, if all biomolecules included in the respective partitioned regions satisfy the specific condition, the state identification unit 109 determines that the biomolecules of the subject organism satisfy the specific condition. Further, if any of the biomolecules included in the respective partitioned regions does not satisfy the specific condition, the state identification unit 109 determines that the biomolecules of the subject organism do not satisfy the specific condition.

In this example, as shown in FIG. 19B, the biomolecule of organism "G" included in Region 20 does not change in amount over time. Also, the biomolecule of organism "G" included in Region 15 increases in amount over time. This change is opposite to the decrease in biomolecule amount shown in statistical information for Region 15 associated with "influenza". Furthermore, the biomolecule of organism "G" included in Region 14 does not change in amount over time. In this case, the state identification unit 109 determines that the biomolecules of the subject organism do not satisfy the specific condition. The state identification unit 109 further identifies the organism "G" as being in a state other than "influenza" associated with the statistical information.

Then, the output unit 110 outputs information on the state identified by the state identification unit 109 to the terminal 30. In this example, the output unit 110 causes a state notification screen 40 to be displayed on the display 31 of the terminal 30, as shown in FIG. 18C. In the present embodiment, the state notification screen 40 includes an organism identification section 41, a state notification section 42, an alert section 45, a detail prompting section 46, a "No" section 47, and a "Yes" section 48.

The organism identification section 41 shows text "Ms. G" including information for identifying the subject organism. The state notification section 42 shows text "Current State" and text "Not influenza," which indicates the state identified by the state identification unit 109.

The alert section 45 shows information for alerting the user to the state of the subject organism. In the present embodiment, the output unit 110 causes the alert section 45 to be displayed on the display 31 in response to a biomolecule included in any one of the partitioned regions in the used distribution information satisfying the change condition. When any biomolecule of the subject organism has changed in amount, the state of the subject organism may have changed accordingly. Thus, in the present embodiment, in response to any biomolecule shown in the used distribution information satisfying the change condition, the server device 10 alerts the user to the state of the subject organism even though no biomolecule of the subject organism satisfies the specific condition.

In this example, as shown in FIG. 19B, the biomolecule of the organism "G" included in Region 15 and shown in the dashed-line portion IV increases in amount over time and satisfies the change condition. Thus, the alert section 45 is displayed on the state notification screen 40. In the illustrated example, the alert section 45 displays text "Caution" and text "The health state may be other than a healthy state."

The detail prompting section 46 shows information for prompting the user to cause the state identification unit 109 to identify a detailed state of the subject organism. In the illustrated example, the detail prompting section 46 shows text "Would you like to identify another state?" In response to the user selecting the "No" section 47, the state identification process ends without identifying a detailed state of the subject organism. In response to the user selecting the "Yes" section 48, the state identification process shown in FIG. 8 is executed, whereby a detailed state of the subject organism is identified and a state notification screen 40 (see FIG. 12) relating to the identified state is displayed on the display 31 of the terminal 30.

As described above, in the present embodiment, in response to a user selecting the type of state of a subject organism to be identified by the state identification unit 109, the information acquisition unit 101 acquires type information indicating the selected type of state. Then, based on the type of state identified by the type information, the target determination unit 108 determines the used distribution information for identifying a state of the subject organism. Further, the state identification unit 109 identifies a state of the subject organism based on a change in one or more biomolecules shown in the used distribution information determined by the target determination unit 108. That is, in the present embodiment, the information acquisition unit 101 acquires item information related to an item corresponding to the state to be identified by the state identification system 1, among a plurality of items categorized corresponding to respective states of an organism. Then, the state identification unit 109 identifies a state of the subject organism based on a change in one or more specific targets associated with respective biomolecules determined according to the item identified by the item information, the change being identified from the used distribution information. Examples of the item information include type information. The information acquisition unit 101 can also be regarded as the item information acquisition unit configured to acquire item information. This feature allows a state of the subject organism to be identified based on one or more biomolecules selected from a plurality of biomolecules according to an item corresponding to the state to be identified for the subject organism.

In the present embodiment, when the used distribution information includes the two sets of subject distribution information shown in FIG. 9A and "influenza" is selected as the type of state to be identified by the state identification unit 109, the state identification unit 109 identifies a state of the subject organism as being "influenza." In this case, the output unit 110 causes the state notification screen 40 shown in FIG. 12 to be displayed on the display 31 of the terminal 30. In the present embodiment, when the used distribution information includes the two sets of subject distribution information shown in FIG. 13A and "effective response to anticancer agent" is selected as the type of state to be identified by the state identification unit 109, the state identification unit 109 identifies a state of the subject organism as being "effective response to anticancer agent." Also, the output unit 110 causes the state notification screen 40 relating to the state identified by the state identification unit 109 to be displayed on the display 31 of the terminal 30. In the present embodiment, when the used distribution information includes the four sets of subject distribution information shown in FIG. 15 and "quality" is selected as the type of state to be identified by the state identification unit 109, the state identification unit 109 identifies a state of the subject organism as being "good quality." Also, the output unit 110 causes the state notification screen 40 relating to the state identified by the state identification unit 109 to be displayed on the display 31 of the terminal 30.

### (EXAMPLE)

An example carried out by the present disclosers will now be described.

The present disclosers created two-dimensional electrophoresis images each showing a plurality of biomolecules of an organism obtained at each of a plurality of time points and, based on the plurality of created images, examined changes over time in the amount of biomolecule shown in the two-dimensional electrophoresis images. In this example, three racehorses were selected as the organisms for examining changes in their biomolecule amount. These three racehorses may be individually referred to as "horse A," "horse B," and "horse C." When these horses for examining changes in their biomolecule amount are described without distinction, they may be referred to as "subject horses." The present disclosers created two-dimensional electrophoresis images showing proteins contained in serum obtained from the subject horses. At the time of serum collection, all the subject horses were two years old. At that time, the weights of horses A, B, and C were 348 kg, 379 kg, and 377 kg, respectively.

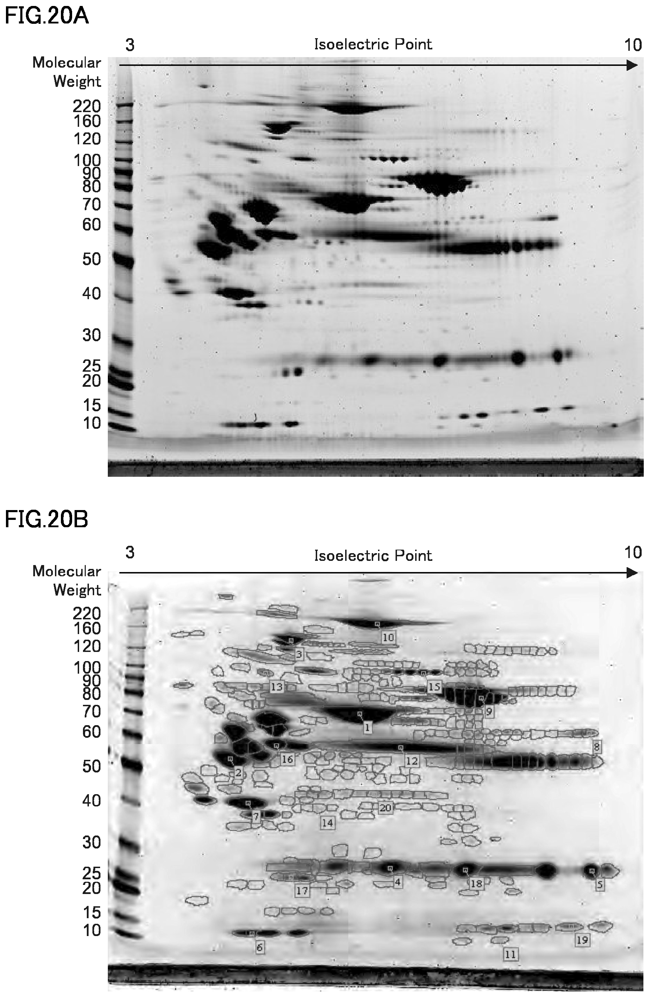

FIG. 20A illustrates a two-dimensional electrophoresis image showing proteins contained in serum obtained from horse A at a certain time point. In this two-dimensional electrophoresis image, a distribution of the amount of protein is shown in a two-dimensional Cartesian coordinate system, in which the horizontal axis represents the isoelectric points of proteins and the vertical axis represents the molecular weights of proteins. The black spots shown in this two-dimensional electrophoresis image each represent a protein. A higher intensity of each black spot indicates a larger amount of the corresponding protein. The present disclosers obtained serum from each subject horse at fifteen time points and created a two-dimensional electrophoresis image for each obtained serum sample. More specifically, each subject horse was infected with an influenza virus, and serum was obtained from the subject horse on each day from the day of infection through day 14. Thereafter, a two-dimensional electrophoresis image was created for each obtained serum sample.

The present disclosers then partitioned the area shown in each created two-dimensional electrophoresis images into a plurality of regions. FIG. 20B illustrates the two-dimensional electrophoresis image of FIG. 20A partitioned into a plurality of regions. In the illustrated example, the area shown in the two-dimensional electrophoresis image is partitioned into 338 regions. Numbers 1 to 20 are assigned to twenty regions, which are examples of the partitioned regions. For each of the created two-dimensional electrophoresis images, the present disclosers partitioned the area into a plurality of regions in the same manner as in the example shown in FIG. 20B.

For each of the created two-dimensional electrophoresis images, the present disclosers determined the amount of protein contained in each partitioned region. The present disclosers calculated statistics for each subject horse based on the determined amount of protein. More specifically, the present disclosers calculated statistics of the amount of protein for each day from the day of infection of the subject horses. The statistics were calculated for each region partitioned in the two-dimensional electrophoresis images. The present disclosers evaluated changes over time in the amount of protein based on the calculated statistics.

### (Comparative Example)

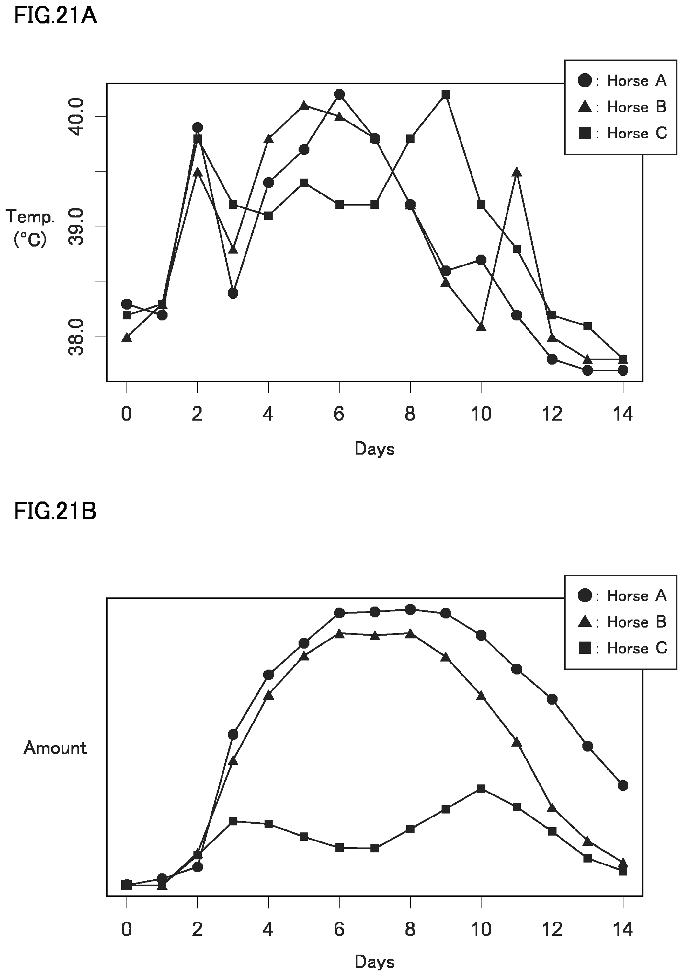

As a comparative example to the above example, the body temperature of each subject horse and the amount of amyloid A therein, which is an example of proteins obtained from the subject horse, were measured on each day from the day of infection with the influenza virus through day 14. FIG. 21A shows the body temperature of each subject horse. In FIG. 21A, the horizontal axis represents the number of days from infection with the influenza virus, and the vertical axis represents the body temperature of each subject horse. In FIG. 21A, the symbols of a black circle each represent horse A, the symbols of a black triangle each represent horse B, and the symbols of a black square each represents horse C. FIG. 21B shows the amount of amyloid A obtained from each subject horse. In FIG. 21B, the horizontal axis represents the number of days from infection with the influenza virus and the vertical axis represents the amount of amyloid A of each subject horse. In FIG. 21B, the symbols of a black circle each represent horse A, the symbols of a black triangle each represent horse B, and the symbols of a black square each represent horse C.

From FIG. 21A, an increase in body temperature was observed in all the subject horses two days after infection with the influenza virus. Thus, a horse is identified as being infected with an influenza virus based on an increase in body temperature two days after the infection. In other words, it takes two days after infection with an influenza virus for a user to identify a horse as being infected with the influenza virus based on body temperature.

From FIG. 21B, an increase in the amount of amyloid A was observed in all the subject horses three days after infection with the influenza virus. Thus, a horse is identified as being infected with an influenza virus based on an increase in the amount of amyloid A three days after the infection. In other words, it takes three days after infection with an influenza virus for a user to identify a horse as being infected with the influenza virus based on the amount of amyloid A. The method described in the comparative example can be regarded as an example of a method for identifying a state of a subject organism using only biomolecules whose functions are known.

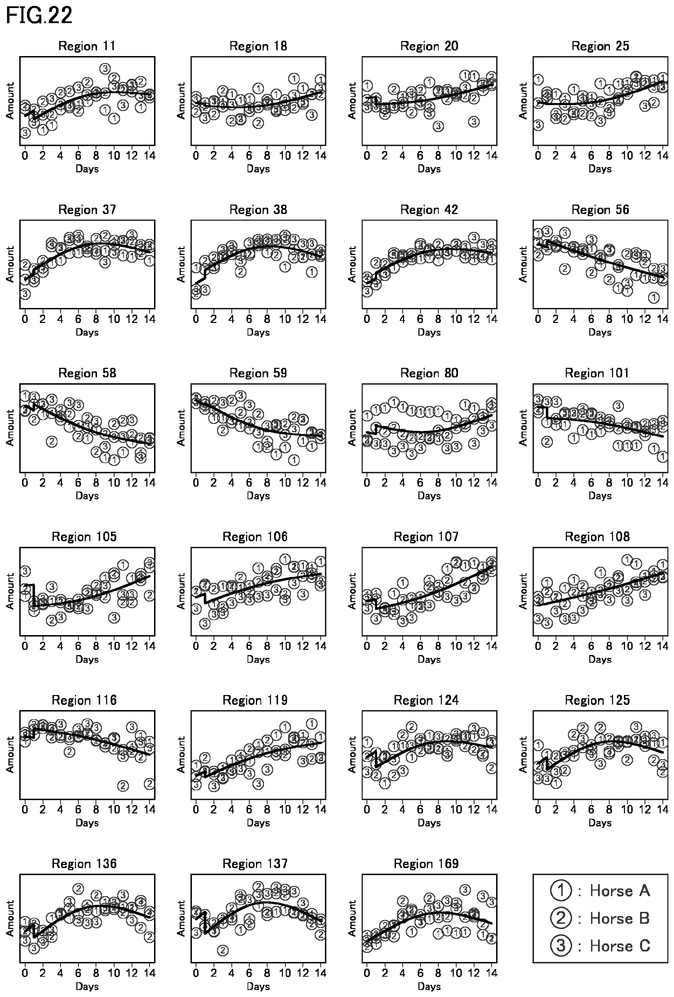

FIG. 22 shows the statistics calculated for the amount of protein contained in the serum of each subject horse. FIG. 22 shows the statistics of the amount of protein contained in partitioned regions where changes in protein amount were observed, among the 338 regions partitioned in the two-dimensional electrophoresis image. In each graph shown in FIG. 22, the horizontal axis represents the number of days from infection with the influenza virus and the vertical axis represents the amount of protein included in each partitioned region in the two-dimensional electrophoresis image. In the illustrated example, for Regions 11, 18, 20, 25, 37, 38, 42, 56, 58, 59, 80, 101, 105, 106, 107, 108, 116, 119, 124, 125, 136, 137, and 169, the protein amount of each subject horse and the statistics of the protein amount are shown for each day from infection with the influenza virus. In each partitioned region in FIG. 22, the reference numeral "1" denotes the protein amount of horse A, the reference numeral "2" denotes the protein amount of horse B, and the reference numeral "3" denotes the protein amount of horse C. In each partitioned region shown in FIG. 22, the solid line represents the statistics calculated for the protein amount of the subject horses.

As shown in FIG. 22, all the subject horses exhibited changes over time in protein amount after infection with the influenza virus. In particular, the statistics for Regions 11, 18, 20, 101, 105, 106, 107, 119, 124, 125, 136, and 137 showed a decrease in protein amount one day after infection with the influenza virus. The statistics for Regions 37, 38, 42, 56, 58, 80, and 116 showed an increase in protein amount one day after infection with the influenza virus. Therefore, based on changes in protein amount shown in FIG. 22 one day after infection with the influenza virus, the horse is identified as being infected with the influenza virus. In other words, it takes one day after infection with an influenza virus for a user to identify a horse as being infected with the influenza virus based on changes in protein amount shown in FIG. 22. In other words, using a two-dimensional electrophoresis image showing biomolecules, a state of an organism can be identified earlier than when the user uses the body temperature of the organism or the amount of amyloid A in the organism. In addition, identifying a state of an organism based on changes in biomolecules shown in a two-dimensional electrophoresis image allows for higher accuracy than identifying a state of the organism based on changes in a single biomolecule. The method described in this example can be regarded as an example of a method for identifying a state of a subject organism using not only biomolecules whose functions are known but also biomolecules whose functions are unknown.

While exemplary embodiments of the present disclosure have been described above, the technical scope of the present disclosure is not limited to the above-described embodiments. It will be apparent from the appended claims that various modifications and improvements to the above embodiments are also included within the technical scope of the present disclosure.

In the above-described embodiments, the state of a subject organism to be identified by the state identification unit 109 varies depending on which used distribution information is used in the state identification process (see FIGS. 9A, 13A, and 15). Here, biomolecules shown in each set of used distribution information are all biomolecules contained in components obtained from each subject organism, and which biomolecules are to be obtained from the subject organism is not predetermined.

In the above-described embodiments, the information acquisition unit 101 acquires a two-dimensional electrophoresis image including information relating to a plurality of biomolecules as the molecular information; however, this is not limiting. For example, the information acquisition unit 101 may acquire, for each biomolecule, molecular information relating to a single biomolecule. In other words, the acquisition of molecular information for a plurality of biomolecules by the information acquisition unit 101 includes both the acquisition of molecular information relating to a plurality of biomolecules and the acquisition of a plurality of pieces of molecular information each corresponding to a single biomolecule.

In the above-described embodiments, information indicating a distribution of physical quantities of biomolecules includes information indicating molecular weights of biomolecules, information indicating isoelectric points of biomolecules, information indicating amounts of biomolecules; however, this is not limiting. Information indicating a distribution of physical quantities of biomolecules may be, for example, information indicating a distribution of electric charges of biomolecules. For example, in a two-dimensional electrophoresis image, a distribution of electric charges of biomolecules may be shown instead of a distribution of isoelectric points of biomolecules. Further, information indicating a distribution of physical quantities of biomolecules may be, for example, information indicating a distribution of hydrophobicity of biomolecules or information indicating three-dimensional structures of biomolecules. In this case, a two-dimensional electrophoresis image may show a distribution of hydrophobicity of biomolecules on one of the x- and y-axes and a distribution of molecular weights of biomolecules on the other. In another example, a two-dimensional electrophoresis image may show three-dimensional structures of biomolecules on one of the x- and y-axes and a distribution of isoelectric points of biomolecules on the other. To further illustrate, any physical properties may be represented on the x- and y-axes in a two-dimensional electrophoresis image, provided that the property represented on the x-axis is different from that represented on the y-axis. In still another example, the biomolecule analysis device 20 may create a three-dimensional electrophoresis image in which three pieces of information indicating different physical properties among the above-described physical properties are respectively represented on the x-, y-, and z- axes. Such a three-dimensional electrophoresis image may also be used to identify a state of an organism.

In the above-described embodiments, distribution information indicates a distribution of biomolecules; however, this is not limiting. For example, information indicating the amount of biomolecule may be created for each isoelectric point or each electric charge of biomolecules. In another example, information indicating the amount of biomolecule may be created for each molecular weight of biomolecules. Thus, the server device 10 may identify a distribution of biomolecules based on information created for each isoelectric point of biomolecules, information created for each electric charge of biomolecules, information created for each molecular weight of biomolecules, or the like.

In the above-described embodiments, the molecular information includes information indicating physical quantities of biomolecules; however, this is not limiting. The molecular information may be, for example, information indicating whether one or more biomolecules are present. In other words, molecular information may be any information relating to biomolecules.

In the above-described embodiments, biomolecules used for statistical calculation by the statistical processing unit 106 are those associated with the same state among individual states; however, this is not limiting. For example, biomolecules used for statistical calculation by the statistical processing unit 106 may be those obtained from a plurality of organisms when the number of days elapsed since the plurality of organisms entered a specific overall state is the same.

In the above-described embodiments, biomolecules used for statistical calculation by the statistical processing unit 106 are those obtained from different organisms; however, this is not limiting. Biomolecules used for statistical calculation by the statistical processing unit 106 may include those obtained from the same organism.

In the above-described embodiments, the detection unit 107 uses, for comparison, two sets of used distribution information corresponding to the closest biomolecule acquisition time points, among three or more sets of used distribution information; however, this is not limiting. The detection unit 107 may use, for comparison, two sets of used distribution information corresponding to biomolecule acquisition time points that are temporally spaced, among three or more sets of used distribution information. For example, the detection unit 107 may use the first-time-point distribution information and the fourth-time-point distribution information shown in FIG. 15 for comparison to detect any change from the biomolecules shown in the first-time-point distribution information to those shown in the fourth-time-point distribution information.

In the above-described embodiments, biomolecules compared by the detection unit 107 and biomolecules determined as comparison targets by the target determination unit 108 are those included in respective partitioned regions having the same coordinates; however, this is not limiting. Biomolecules compared by the detection unit 107 and biomolecules determined as comparison targets by the target determination unit 108 may be those included in respective partitioned regions having different coordinates, provided that these biomolecules are included in mutually corresponding partitioned regions. In this case, the detection unit 107 and the target determination unit 108 may identify such biomolecules included in mutually corresponding partitioned regions on condition that the biomolecules to be compared are shown within a predetermined coordinate range. Alternatively, the detection unit 107 and the target determination unit 108 may identify such biomolecules included in mutually corresponding partitioned regions based on positional relationships between biomolecules to be compared and other biomolecules in distribution information.

In the above-described embodiments, the state identification unit 109 identifies, as a state of a subject organism, an overall state associated with statistical information indicating one or more biomolecules satisfying the sameness/similarity condition; however, this is not limiting. The state identification unit 109 may identify, as a state of a subject organism, one of individual states associated with statistical information indicating one or more biomolecules satisfying the sameness/similarity condition.

In the above-described embodiments, the state identification unit 109 identifies a state of a subject organism according to one or more changes in the amount of biomolecule in the subject organism; however, this is not limiting. For example, the detection unit 107 may detect one or more changes in molecular weight, changes in isoelectric point, changes in electric charge, or changes in the presence or absence of biomolecules, based on molecular information relating to the subject organism. Then, the state identification unit 109 may extract molecular information relating to one or more comparative organism that exhibited one or more changes corresponding to the changes detected by the detection unit 107 and may identify, as a state of the subject organism, a state of the comparative organism associated with the extracted molecular information. In other words, changes in biomolecules used to identify a state of a subject organism are not limited to changes in the amount of biomolecule.

In the above-described embodiments, the server device 10 acquires, for a single organism, a plurality of pieces of molecular information relating to biomolecules obtained at different time points and identify one or more changes in biomolecules over time based on the acquired plurality of pieces of molecular information; however, this is not limiting. The server device 10 may acquire molecular information indicating one or more changes in biomolecules over time, such as information indicating one or more changes in biomolecules during a specific period. Examples of changes in biomolecules include changes in a specific physical quantity of biomolecules and changes in the presence or absence of biomolecules. Examples of the specific physical quantity include amount, molecular weight, isoelectric point, and electric charge. The server device 10 may identify one or more changes in biomolecules over time based on the acquired molecular information. In other words, molecular information may be any form or type of information that allows the server device 10 to identify one or more changes in biomolecules.

In the above-described embodiments, the change condition is defined based on changes in biomolecules; however, this is not limiting. For example, the change condition may be defined such that no change occurs in biomolecules. In one example, the change condition may be defined such that a difference between the amounts of two biomolecules is less than or equal to a predetermined value. The state identification unit 109 may then extract molecular information indicating biomolecules of one or more comparative organisms corresponding to biomolecules satisfying the change condition and may identify, as a state of the subject organism, a state associated with the extracted molecular information. In one example, in the used distribution information shown in FIG. 9B, when the biomolecule included in Region 20 shows no change in amount and thus satisfies the change condition and the biomolecule included in Region 15 also shows no change in amount and thus satisfies the change condition, the state of the subject organism may be identified as being another state that is neither "influenza" nor "COVID-19." Examples of such other states include a healthy state of the subject organism.

The above-described molecular information that enables the identification of a state of a biomolecule over time, from before a specific action acts on the subject organism to after the specific action has acted thereon includes not only information enabling the identification of a change in the biomolecule over time but also information enabling the identification of absence of such change in the biomolecule.

The detection unit 107 may detect a degree of change in a biomolecule. More specifically, the detection unit 107 may detect, from each set of used distribution information, which of a plurality of levels a degree of change in a biomolecule included in each partitioned regions corresponds to. The state identification unit 109 may then extract distribution information relating to one or more comparative organisms indicating a change corresponding to the level detected by the detection unit 107 and may identify, as a state of the subject organism, a state associated with the extracted distribution information.

In the above-described embodiments, the state identification unit 109 compares each detected biomolecule with biomolecules shown in statistical information; however, this is not limiting. For example, the state identification unit 109 may compare each detected biomolecule with biomolecules shown in comparative distribution information relating to a comparative organism. When one or more biomolecules shown in the comparative distribution information satisfy the sameness/similarity condition, the state identification unit 109 may identify, as a state of the subject organism, a state associated with this comparative distribution information.

In the above-described embodiments, the subject organism is different from comparative organisms; however, this is not limiting. The subject organism may be the same as one of the comparative organisms.

In the above-described embodiments, the state identification unit 109 identifies a state of a subject organism by comparing biomolecules of the subject organism with those of comparative organisms having the same attribute as the subject organism; however, this is not limiting. The state identification unit 109 may identify a state of a subject organism by comparing biomolecules of the subject organism with those of one or more comparative organisms having an attribute different from that of the subject organism. For example, the state identification unit 109 may identify a state of a subject organism by comparing biomolecules of a human as the subject organism with those of horses as comparative organisms. In this case, the server device 10 may identify characteristics of physical quantities of biomolecules for each attribute of the organisms and, based on the identified characteristics, correct the physical quantities of biomolecules shown in comparative molecular information for each attribute of the comparative organisms. The server device 10 may then identify a state of a subject organism by comparing biomolecules of the subject organism with the corrected biomolecules in the comparative molecular information.

The state identification unit 109 may identify a state of a subject organism based on results of machine learning. The state identification unit 109 learns a relationship between a distribution of biomolecules and a state of an organism using, as training data, distribution information and individual state information in which distributions of the amount of biomolecule shown in two-dimensional electrophoresis images are associated with respective states of organisms at time points when the biomolecules shown in the two-dimensional electrophoresis images were obtained therefrom. The state identification unit 109 creates, based on learning results, a trained model that takes distribution information as input and outputs individual state information. The state identification unit 109 may then identify a state of a subject organism by applying the trained model to distribution information of the subject organism. In other words, the state identification unit 109 may identify a state of a subject organism from a distribution pattern of biomolecules shown in distribution information relating to the subject organism, through pattern recognition where a distribution of biomolecules shown in input distribution information is associated with individual state information. In this manner, the state identification unit 109 may identify a state of a subject organism without comparing biomolecules of the subject organism with other biomolecules. The biomolecules recognized by the state identification unit 109 through pattern recognition in distribution information can be regarded as a target associated with biomolecules that satisfies a condition defined for changes in biomolecules identified from molecular information. The condition defined for changes in biomolecules identified from molecular information is such that one or more biomolecules identified from distribution information are recognized by the state identification unit 109 through pattern recognition. The target associated with biomolecules satisfying the condition is the distribution of biomolecules shown in the distribution information.

In the above-described embodiments, the server device 10 and the biomolecule analysis device 20 are separately provided in the state identification system 1; however, this is not limiting. For example, a device integrating the server device 10 and the biomolecule analysis device 20 may be provided in the state identification system 1. In other words, a single device in the state identification system 1 may perform analysis of biomolecules, creation of molecular information, and identification of a state of a subject organism.

In the above-described embodiments, individual state information and overall state information are input by a user operating the terminal 30 or the server device 10; however, this is not limiting. For example, an organism may be captured using an imaging device such as a video camera. Further, the organism appearing in the captured video may be analyzed using an analysis device configured to analyze organisms, and individual state information and overall state information each indicating a state of the organism may be created based on the analysis results and transmitted to the information acquisition unit 101. In this manner, the information acquisition unit 101 may acquire information from means other than the terminal 30.

In the present embodiment, the server device 10 identifies a state of a subject organism; however, this is not limiting. For example, the terminal 30 may have functions of the server device 10. In other words, the terminal 30 may include functions corresponding to the information acquisition unit 101, the storage unit 102, the region recognition unit 103, the partitioning unit 104, the amount determination unit 105, the statistical processing unit 106, the detection unit 107, the target determination unit 108, the state identification unit 109, and the output unit 110 of the server device 10.

A program for implementing the embodiments of the present disclosure may be provided on a computer-readable recording medium, such as a magnetic recording medium (e.g., a magnetic tape or a magnetic disk), an optical recording medium (e.g., an optical disk), a magneto-optical recording medium, and a semiconductor memory. The program may also be provided via a communication means such as the Internet.

While multiple embodiments have been described above, features from one embodiment may be replaced with or incorporated into those of another embodiment.

While the present disclosure has been described with reference to embodiments, it is to be understood that the disclosure is not limited to the disclosed embodiments. The scope of the following claims is to be accorded the broadest interpretation so as to encompass all such modifications and equivalent structures and functions.

Various embodiments have been described in detail above but it will be understood that the present disclosure is not limited to these embodiments and encompasses all modifications, variants, alternatives and equivalents falling within the scope of the appended claims.

## Claims

1. An information processing system (1) comprising:
an acquisition unit (101) configured to acquire molecular information relating to biomolecules of an organism for a plurality of biomolecules; and
an identification unit (109) configured to identify a state of the organism according to a target associated with a biomolecule among the plurality of biomolecules, the target satisfying a condition defined for changes in biomolecules identified from the molecular information.

2. The information processing system according to claim 1, wherein the target is a portion that is included in the biomolecule and satisfies the condition.

3. The information processing system according to claim 1, wherein
the molecular information includes information on biomolecules in each of a plurality of segments defined according to physical quantities of biomolecules, and
the condition is defined for each of the plurality of segments.

4. The information processing system according to claim 3, wherein
the molecular information includes information on presence of biomolecules at a plurality of time points in each of the plurality of segments, and
the plurality of segments include a segment in which no biomolecule of the organism is present at at least one of the plurality of time points.

5. The information processing system according to claim 1, wherein
the plurality of biomolecules include a first biomolecule and a second biomolecule different from the first biomolecule, and
the identification unit is configured to identify the state in response to both a specific target associated with the first biomolecule and a specific target associated with the second biomolecule in the organism satisfying the condition.

6. The information processing system according to claim 5, wherein
an increase in a specific physical quantity of the target associated with the first biomolecule satisfies the condition, and
a decrease in the physical quantity of the target associated with the second biomolecule satisfies the condition.

7. The information processing system according to claim 5, wherein
the plurality of biomolecules further include a third biomolecule different from both the first biomolecule and the second biomolecule, and
the identification unit is configured to:
identify the state as being a first state in response to the target associated with the first biomolecule and the target associated with the second biomolecule in the organism satisfying the condition and a specific target associated with the third biomolecule in the organism not satisfying the condition; and
identify the state as being a second state in response to the target associated with the first biomolecule and the target associated with the third biomolecule in the organism satisfying the condition and the target associated with the second biomolecule in the organism not satisfying the condition.

8. The information processing system according to claim 1, wherein the identification unit is configured to identify the state in response to both a first portion and a second portion of a specific biomolecule among the plurality of biomolecules in the organism satisfying the condition.

9. The information processing system according to claim 8, wherein
an increase in a specific physical quantity of the first portion satisfies the condition, and
a decrease in the physical quantity of the second portion satisfies the condition.

10. The information processing system according to claim 1, wherein
the molecular information includes information that enables identification of a state of biomolecules over time, from before a specific action acts on the organism to after the action has acted thereon, and
the identification unit is configured to:
identify the state as being a first state relating to the action in response to a specific target associated with a specific biomolecule among the plurality of biomolecules satisfying the condition; and
identify the state as being a second state relating to the action in response to the target associated with the specific biomolecule not satisfying the condition.

11. The information processing system according to claim 10, wherein
the plurality of biomolecules include a first biomolecule and a second biomolecule different from the first biomolecule,
the specific biomolecule is the first biomolecule, and
the identification unit is configured to:
identify the state as being the first state in response to the target associated with the first biomolecule satisfying the condition and a specific target associated with the second biomolecule not satisfying the condition; and
identify the state as being the second state in response to the target associated with the first biomolecule not satisfying the condition and the target associated with the second biomolecule satisfying the condition.

12. The information processing system according to claim 10, wherein
the plurality of biomolecules include a first biomolecule and a second biomolecule different from the first biomolecule,
the specific biomolecule is the first biomolecule, and
the identification unit is configured to:
identify the state as being the first state in response to both the target associated with the first biomolecule and a specific target associated with the second biomolecule satisfying the condition; and
identify the state as being the first state in response to the target associated with the first biomolecule not satisfying the condition and the target associated with the second biomolecule satisfying the condition.

13. The information processing system according to claim 1, wherein
the molecular information includes information that enables identification of changes in biomolecules during a first period and information that enables identification of changes in biomolecules during a second period subsequent to the first period, and
the identification unit is configured to identify the state according to a target associated with a biomolecule that satisfies the condition for the changes during the first period and a target associated with a biomolecule that satisfies the condition for the changes during the second period.

14. The information processing system according to claim 13, wherein the target satisfying the condition for the changes during the first period is the same as the target satisfying the condition for the changes during the second period.

15. An information processing system comprising:
an acquisition unit configured to acquire molecular information relating to biomolecules of an organism for a plurality of biomolecules; and
an output unit configured to output information relating to a state of the organism, the information corresponding to a target associated with a biomolecule satisfying a condition defined for changes in biomolecules identified from the molecular information.

16. An identification system for identifying a state of an organism, comprising:
a molecular information acquisition unit configured to acquire molecular information relating to biomolecules of the organism for a plurality of biomolecules;
an item information acquisition unit configured to acquire item information relating to a target item to be identified by the identification system, among a plurality of items categorized corresponding to respective states of the organism; and
an identification unit configured to identify the state based on a change in a target associated with a biomolecule determined according to the item identified from the item information, the change being identified from the molecular information.

17. A program for causing a computer to implement:
a function of acquiring molecular information relating to biomolecules of an organism for a plurality of biomolecules; and
a function of identifying a state of the organism according to a target associated with a biomolecule among the plurality of biomolecules, the target satisfying a condition defined for changes in biomolecules identified from the molecular information.
